# NEUE EUROPÄISCHE PATENTSCHRIFT

(11) **EP 1 982 655 B2**
(45) Veröffentlichungstag und Bekanntmachung des Hinweises auf die Entscheidung über den Einspruch: **07.12.2022**
(45) Hinweis auf die Patenterteilung: 05.06.2013
(21) Anmeldenummer: 07106278.0
(22) Anmeldetag: 16.04.2007
(51) Int. Cl.: A61B 17/00, A61B 17/12, A61F 2/01

(54) **Occluder zum Verschliessen eines Herzohres und Herstellungsverfahren dafür**
Occluder to seal an atrial appendage and method of manufacture thereof
Dispositif d'occlusion d'une oreillette cardiaque et sa méthode de manufacture

(43) Veröffentlichungstag der Anmeldung: 22.10.2008
(60) Teilanmeldung: 11191896.7
(73) Patentinhaber: Occlutech Holding AG, 8200 Schaffhausen (CH)
(72) Erfinder: Figulla, Prof. Hans-Reiner, 07749, Jena (DE); Schräder, Prof. Dr. med R., 60596, Frankfurt (DE); Krizanic, Dr. Florian, 07743, Jena (DE); Moszner, Dr. Robert, 07639, Bad Klosterlausnitz (DE); Moszner, Friedrich, 99441, Hohlstedt (DE); Schmidt, Dr. Kathrin, 07768, Kahla (DE); Ottma, Rüdiger, 99441, Großschwabhausen (DE)
(74) Vertreter: KIPA AB

(56) Entgegenhaltungen:
- WO-A-02/071977
- DE-B3- 10 338 702
- US-A- 5 944 738
- US-A1- 2006 247 680
- US-B1- 6 168 622

## Beschreibung

### Gebiet der Erfindung

Die vorliegende Erfindung betrifft ein selbst expandierbares Occlusionsinstrument zum Verschließen eines Herzohres, bestehend aus einem Geflecht dünner Drähte, oder Fäden, welches mittels eines Formgebungs-, Umformungs- und/oder Wärmebehandlungsverfahrens eine geeignete Formgebung erhält, wobei das Occlusionsinstrument einen vorderseitigen proximalen Retentionsbereich und einen rückseitigen distalen Retentionsbereich aufweist, und wobei in dem proximalen Retentionsbereich die Enden der Drähte oder Fäden in einer Fassung aber auch in einer zweiten Lösung ohne Fassung zusammenlaufen. Des Weiteren weist das Occlusionsinstrument einen Mittenbereich zwischen dem proximalen und dem distalen Retentionsbereich auf. Das Occlusionsinstrument ist dabei so ausgeführt, dass es in einem zusammengefalteten Zustand mittels eines Katheters in den Körper eines Patienten minimal-invasiv einführbar und in dem Herzohr des Patienten positionierbar ist. Des Weiteren betrifft die Erfindung Verfahren zur Herstellung eines solchen Occlusionsinstruments.

### Hintergrund der Erfindung

Ein derartiges Occlusionsinstrument ist zumindest teilweise dem Prinzip nach aus der Medizintechnik bekannt. Beispielsweise ist in der DE 10 338 702 vom 22. August 2003 ein Occlusionsinstrument zur Behandlung von Septumdefekten bekannt, welches aus einem Geflecht dünner Drähte bzw. Fäden besteht und mittels eines Umformungs- und Wärmebehandlungsverfahrens eine geeignete Formgebung erhält. Das bekannte Occlusionsinstrument weist einen distalen Retentionsbereich, welcher besonders flach ausgeprägt ist, einen proximalen Retentionsbereich und einen zylindrischen Steg zwischen dem proximalen und dem distalen Retentionsbereich auf. Am proximalen Retentionsbereich laufen die Enden der das Geflecht bildenden Drähte in einer Fassung zusammen. Dabei ist vorgesehen, dass die beiden Retentionsbereiche des bekannten Occlusionsinstruments durch einen meist intravaskulären Operationseingriff beiderseits eines zu verschließenden Shuntes in einem Septum zur Anlage kommen, während der Steg durch den Shunt hindurchläuft. Das Occlusionsinstrument ist angepasst, um eine Öffnung mit dynamischem Blutfluss permanent zu verschließen, und weist dazu eine spezielle Konstruktion auf und ist nicht für Herzohrocclussionen geeignet.

Ähnliche Instrumente sind in der Occlutech-Patentanmeldung WO 2007 054116.

Wenn der Patient unter einem so genannten Vorhofflimmern des Herzens leidet, treten besondere emboliebedingte Probleme in Erscheinung. Hierbei handelt es sich um eine frequente Erregung der Vorhöfe des Herzens, die zu keiner Kontraktion der Vorhöfe führt. Folge dieses Kontraktionsverlustes der Vorkammern des Herzens ist es, dass eine wirksame Durchwirbelung und Durchmischung des Blutes ausbleibt und sich Tromben im Vorhof bilden können. Ein erhebliches Risiko bei der Vorhoftrombenbildung infolge Vorhofflimmerns besteht darin, dass solche Tromben mit dem Blutstrom mitgerissen werden können und in die arterielle Zirkulation gelangen. Folgen dieser Embolisation sind insbesondere Schlaganfälle, die in etwa 5% pro Jahr bei Patienten mit Vorhofflimmern auftreten, falls nicht durch eine chronische Behandlung eine Geringungshemmung des Blutes mit so genannten Dicumerolen durchgeführt wird. Eine Herbeiführung der Geringungshemmung des Blutes mit so genannten Dicumerolen ist allerdings ebenfalls nicht risikolos. Nebenwirkungen der Behandlungen mit Dicumerolen sind vermehrte Blutungen, sodass Kontraindikationen für diese Behandlung bei ca. 20% der Patienten mit Vorhofflimmern besteht und die Patienten somit aufgrund von einer Risikoabwägung Blutung/Schlaganfall das Risiko eines Schlaganfalls in Kauf genommen wird.

Tromben im Vorhof des Herzens entstehen in überwiegender Mehrzahl in den so genannten Herzohren. Die Herzohren sind Ausstülpungen an den Vorhöfen des menschlichen Herzens. Das rechte Herzohr liegt neben der aufsteigenden Aorta, und das linke Herzohr neben der großen Lungenarterie. Dabei ist das linke Herzohr bei Patienten mit Vorhofflimmern der häufige Entstehungsort für Blutgerinnsel, die zu einem Schlaganfall führen können.

Aufgrund der im Zusammenhang mit der zuvor beschriebenen Vorhoftrombenbildung genannten Risiken und Probleme liegt der vorliegenden Erfindung die Aufgabe zugrunde, ein Occlusionsinstrument anzugeben, mit dem das Herzohr des linken Vorhofs verschlossen werden kann, um eine Trombusbildung mit dem Risiko eines Schlaganfalls erheblich zu reduzieren. Insbesondere soll ein Occlusionsinstrument angegeben werden, mit dem das Schlaganfallrisiko auch bei solchen Patienten reduziert werden kann, bei denen eine Gerinnungshemmung mit Dicumerolen (so genannte Antikoagulation) aufgrund von Blutungsneigungen kontra indiziert ist. Ebenso sollte das Occlusionsinstrument sicher im Herzohr zu verankern sein, ohne sich zu lösen.

Nach dem Stand der Technik existieren verschiedene Lösungen, welche im Wesentlichen Teilgeflechte mit gestängeartigen Bauelementen kombinieren, teilweise werden dabei auch gelaserte Konstruktionen in dieser Kombination eingesetzt, das betrifft solche Erfindungen wie: EP 1417933A1; US 7,169,164B2; US 2005/0234543A1; US 6, 689, 150B1; US 6, 152, 144.

Diese Erfindungen haben unter anderem den Nachteil, dass für die Implantation relativ große Einführschleusen mit bis zu 14 F Innendurchmesser benötigt werden.

Weitere Erfindungen wie von Amplatz WO 2005 099 365, nach der PLAATO-Methode (PLAATO = Percutaneus Left Atrial Appendage Transcatheter) und Watchman-Occluder beherrschen nicht die korrekte vorgegebene Positionierung. Bei dem System PLAATO besteht die Gefahr der Perforation mit dem Herzbeutel, außerdem wurde PLAATO ebenfalls mit einer 14F-Schleuse implantiert.

WO 02/071977 beschreibt implantierbare Vorrichtungen gemäß dem Oberbegriff des Anspruchs 1, beispielsweise bestehend aus einer geflochtenen Grundstruktur mit daran befestigter proximaler Abdeckstruktur. Die Vorrichtungen sind jedoch verbesserungswürdig mit Hinsicht auf deren zuverlässige Besfestigung im Herzen nach Implantation.

Eine weitere Anmeldung von Occlutech betrifft die Erfindung eines einnabigen Herzohroccluders unter Verwendung eines Fixiermittels zum Ausbilden einer kraftschlüssigen Verbindung zwischen dem Geflecht eines Occlusionskörpers und der Herzohrwandung, PCT/EP 2006/005292 vom 02.06.2006. Dabei ist das Fixiermittel ausgelegt, nach dem Applizieren insbesondere in einer kontrollierten Weise zu einem flexiblen unlöslichen Produkt auszuhärten, um somit eine dauerhafte und feste Verankerung zwischen dem Geflecht des Occlusionskörpers und der Herzohrwandung herzustellen. Erfindungsgemäß ist vorgesehen, dass der distale Retentionsbereich einen Kugelbereich aufweist, der im expandierten Zustand des Occlusionsinstruments in dem zu verschließenden Herzohr an den Innenwandungen des Herzohres zur Anlage kommt und mit den Innenwandungen des Herzohres eine kraftschlüssige Verbindung bildet, um derart das implantierte und expandierte Occlusionsinstrument im Herzohr zu halten, wobei der proximale Retentionsbereich des Occlusionsinstruments die Öffnung des Herzohres verschließt.

### Zusammenfassung der Erfindung

Aufgabe der Erfindung ist es, die oben genannten Nachteile der herkömmlichen Verfahren und Vorrichtungen zu überwinden und eventuell eine möglichst Kosten senkende und patientenfreundliche sowie pateintensichere Lösung anzugeben.

Die oben genannte Aufgabe wird mit einem selbst expandierbaren Occlusionsinstrument zum Verschließen eines Herzohres gelöst, wobei das Occlusionsinstrument aus einem Geflecht dünner Drähte, Litzen oder Fäden besteht, welches mittels eines Formgebungs-, Umformungs- und/oder Wärmebehandlungsverfahrens eine geeignete Formgebung erhalten hat gemäß Anspruch 1

Bevorzugte Weiterentwicklungen der Erfindung bezüglich des Occlusionsinstruments sind in den Unteransprüchen angegeben.

### Kurzbeschreibung der Zeichnungen

Im Folgenden werden bevorzugte Ausführungsbeispiele des erfindungsgemäßen Occlusionsinstruments anhand der Zeichnungen näher erläutert. Es zeigen:
Fig. 1 Eine Seitenansicht eines Herzohroccluders mit einer Fassung;
Fig. 2 Der Herzohroccluders nach Fig. 1 nach Implantation im linken Herzohr (Englisch Left Atrial Appendage - LAA), angrenzend zum linken Vorhof;
Fig. 3, 4 Kugelgeflecht, kugel- und ballonförmig zur Herstellung eines Herzohroccluders nach Fig. 1;
Fig. 5 Eine Seitenansicht eines Herzohroccluders ohne Fassung;
Fig. 6 Die Form nach Fig. 5 im Anschluss an eine Implantation im linken Herzohr des linken Vorhofes;
Fig. 7 Kugelgeflecht aus einem Drahtstück ohne Fassung;

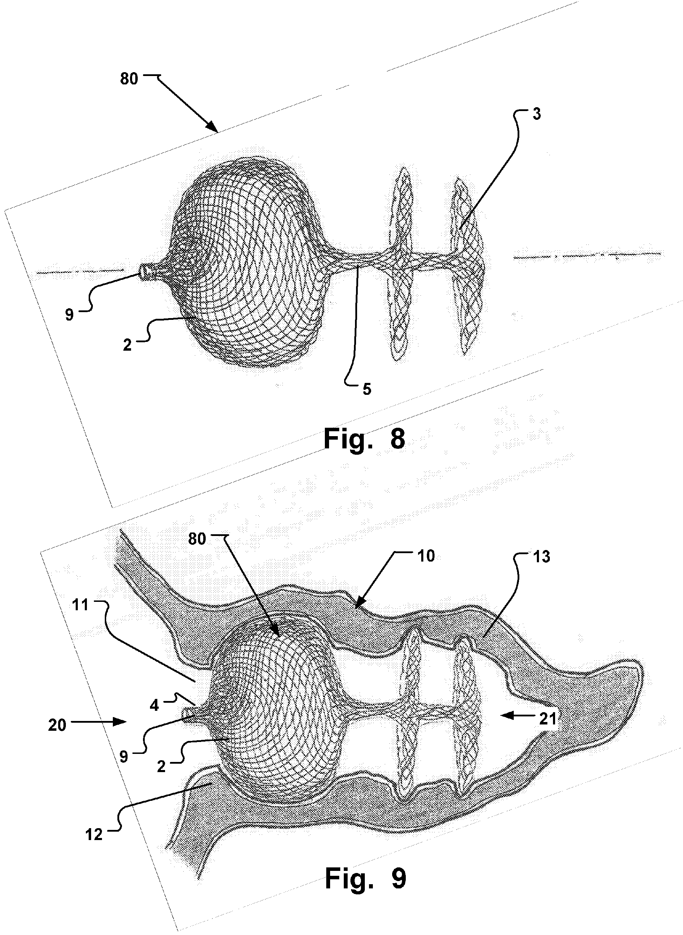

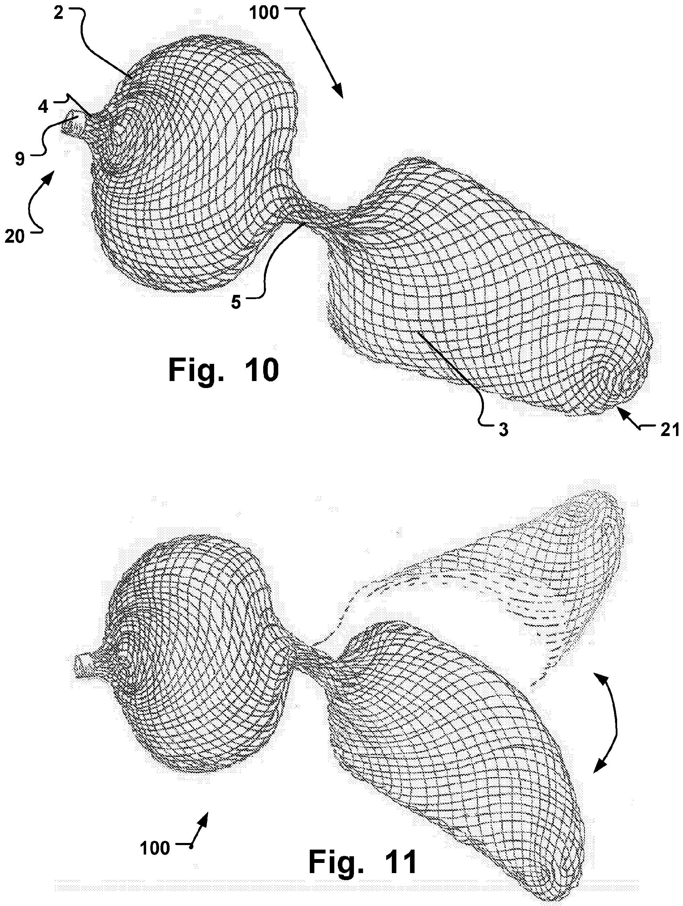

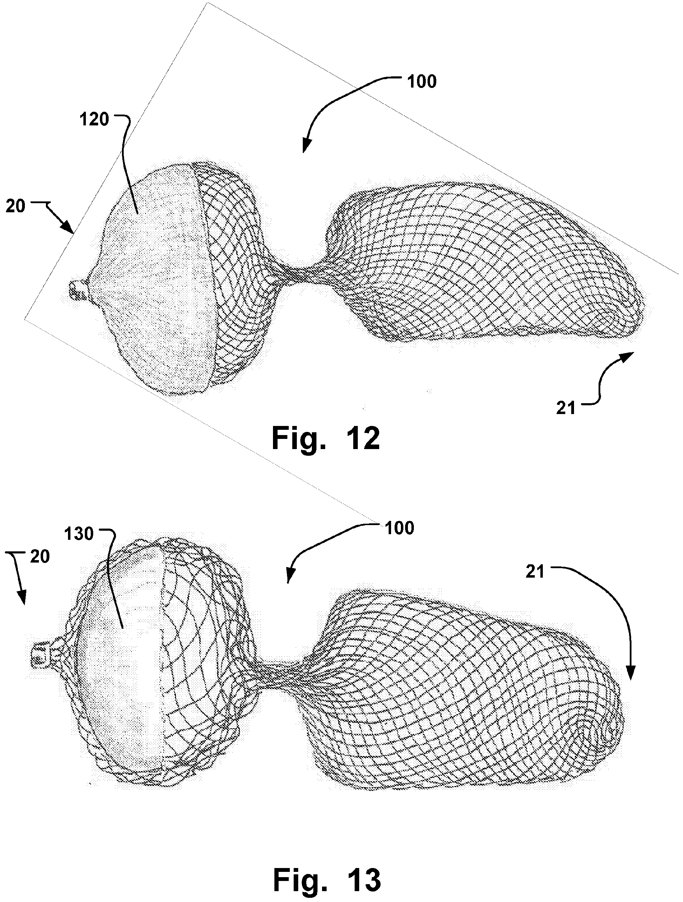

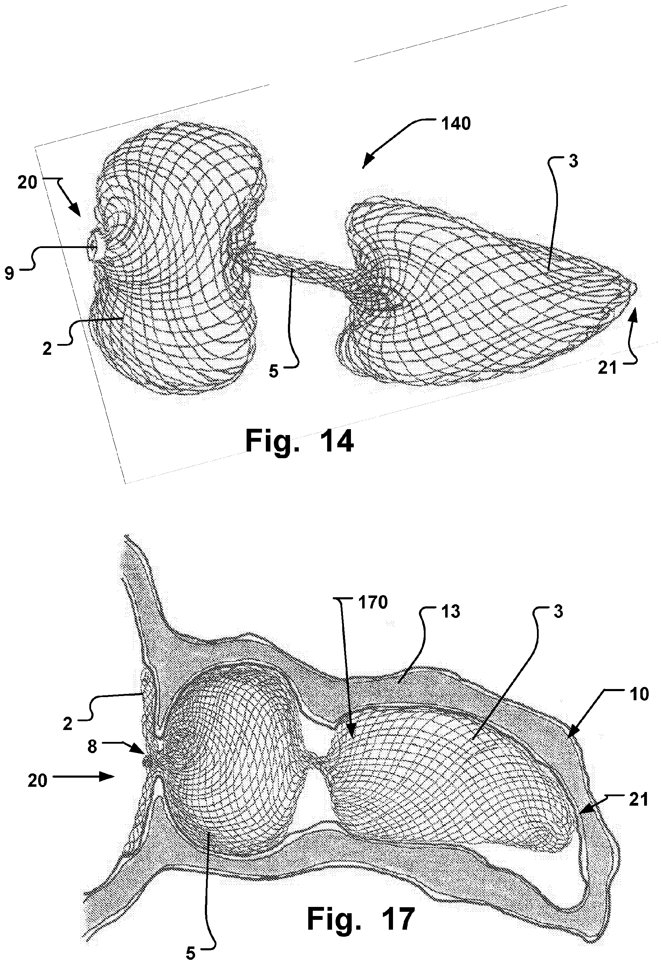

Fig. 8-14: gelöscht
Fig. 15 Fünfte Variante einer Ausführungsform eines Herzohroccluders mit einer flachen Doppelscheibe am proximalen Ende;
Fig. 16 Die Ausführungsform nach der fünften Variante im Anschluss der Implantation in das linke Herzohr;

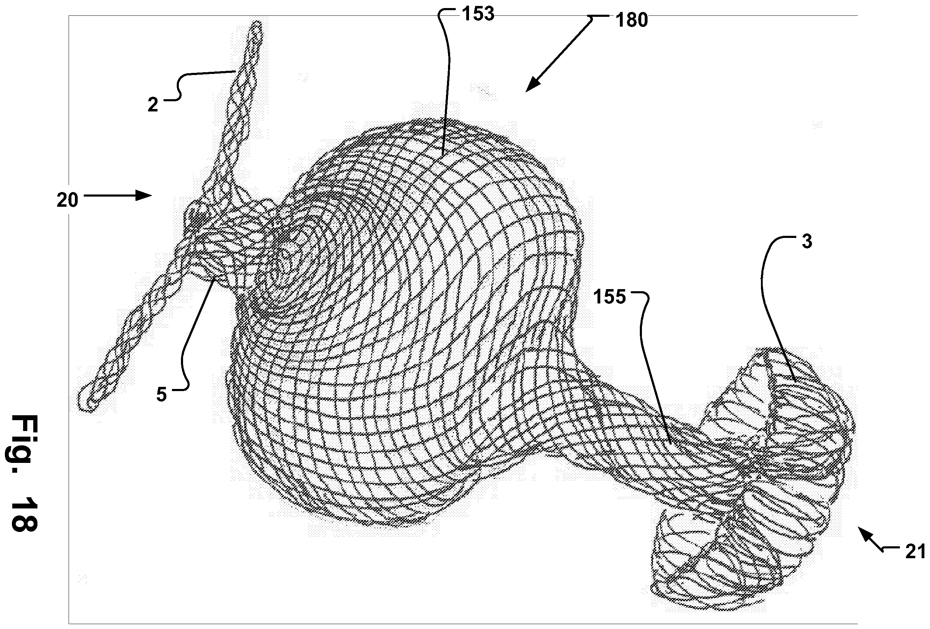

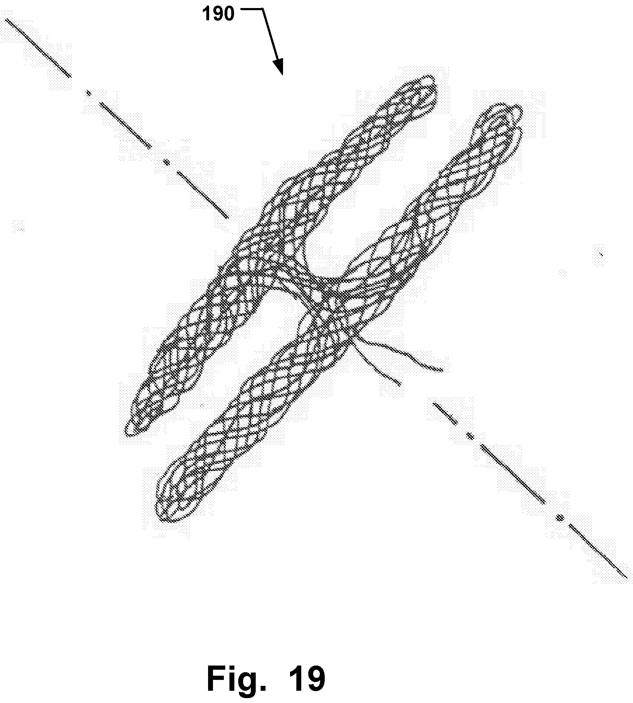

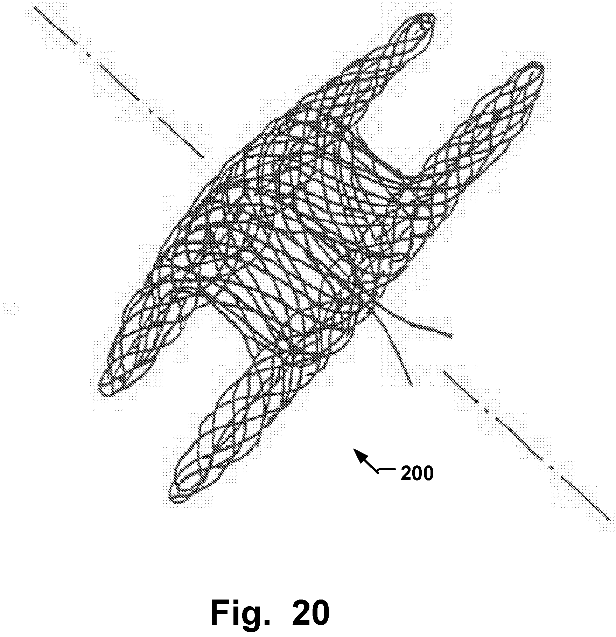

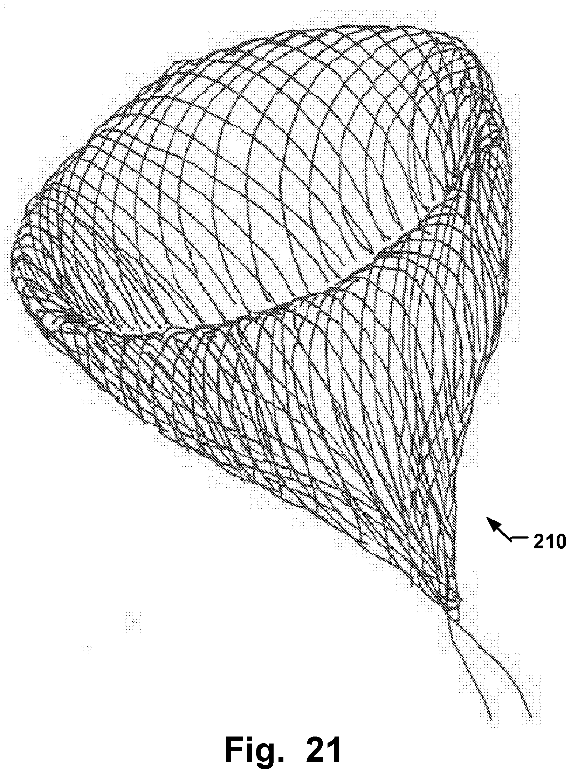

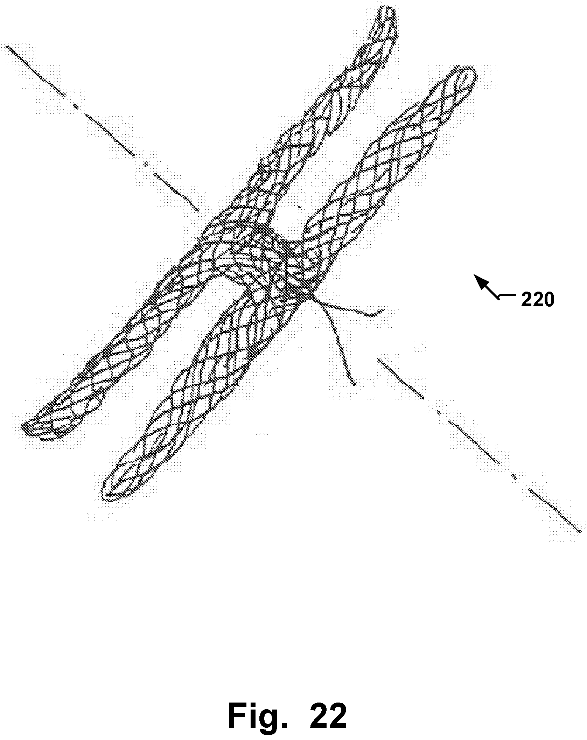

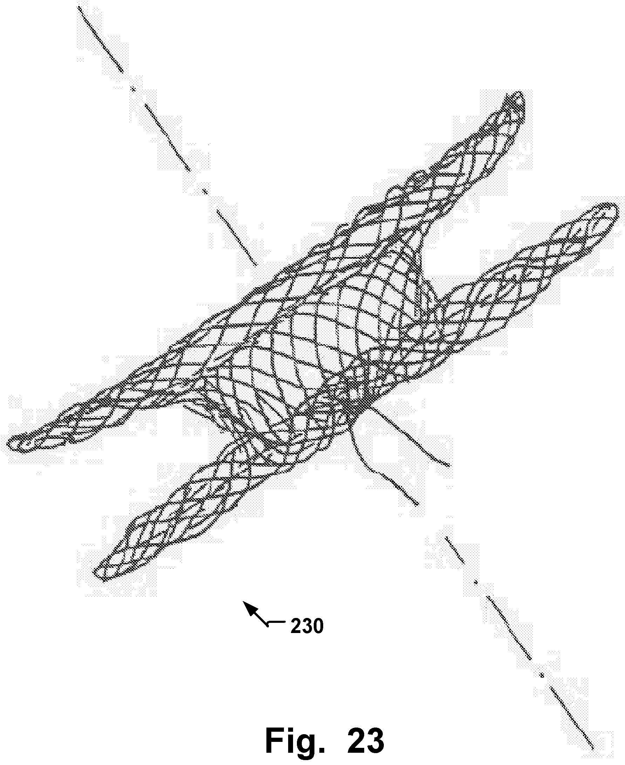

Fig. 17-23: gelöscht.
Fig. 24 Darstellung eines Herzohres.

### Ausführliche Beschreibung der Erfindung

Während die Erfindung nachstehend in einer Ausführungsform zum Durchführen der Erfindung beschrieben werden wird, versteht der Fachmann, dass diese Ausführungsform nur einen illustrative, nicht begrenzende Beispiel von vielfältigen Formen, welche die vorliegende Erfindung einnehmen kann, darstellt.

Selbstverständlich sind hier aber auch andere, wie auch immer geartete und anwendungsspezifische Formgebungen des Occlusionsinstrumtents denkbar. Die hier dargestellte Formgebung dient lediglich zur Beschreibung von einer bevorzugte Ausführungsform des Occlusionsinstruments und soll insbesondere nicht den Schutzumfang der Erfindung in irgendeiner Weise einschränken.

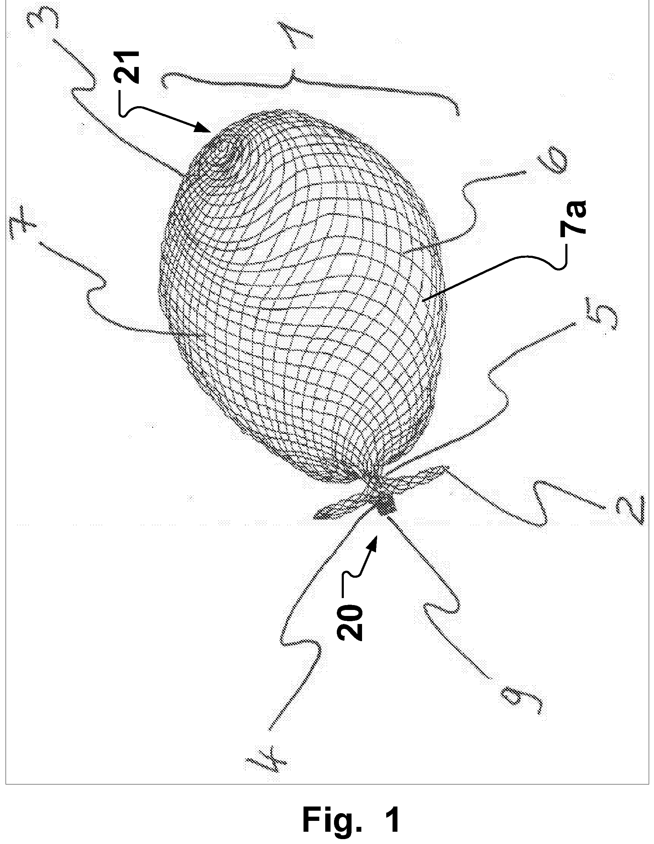

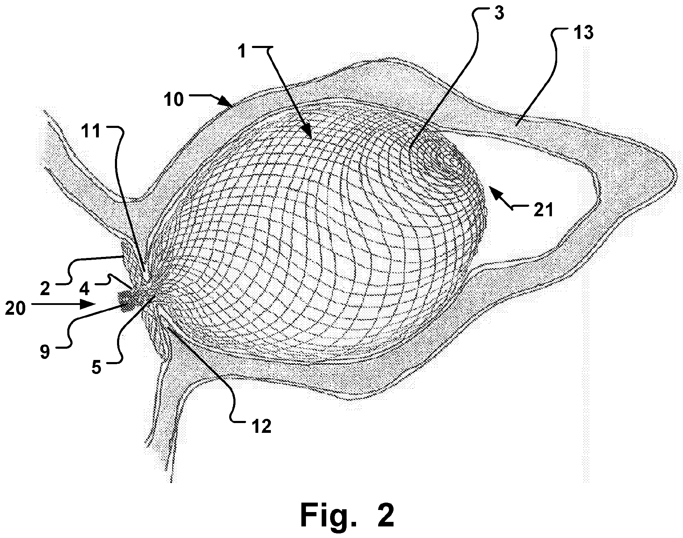

Fig. 1 zeigt eine Seitenansicht eines nicht erfindungsgemäßen Ausführungsform selbst expandierbaren Occlusionsinstruments. In der Fig. 2 sehen wir eine Schnittdarstellung im Bereich linker Vorhof/linkes Herzohr mit implantiertem Herzohroccluder mit einer proximalen Fassung.

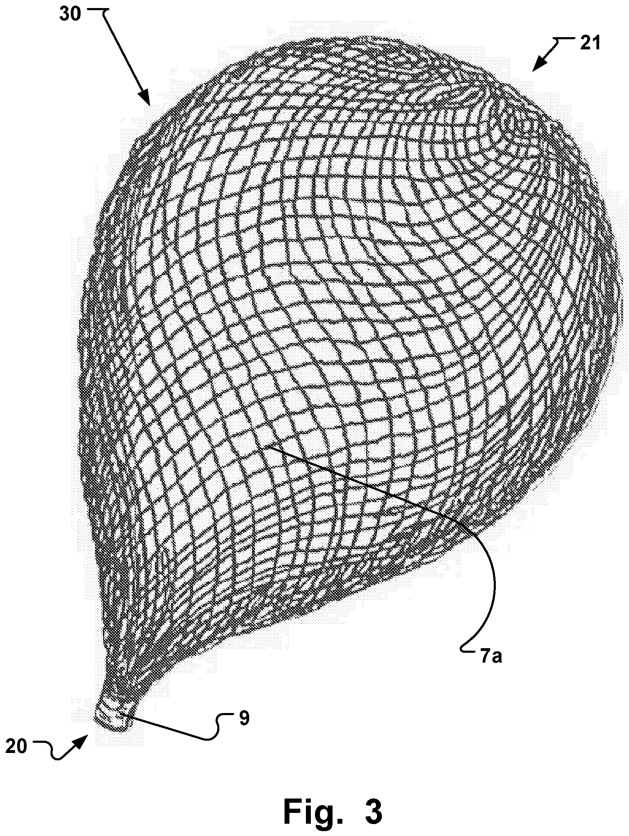

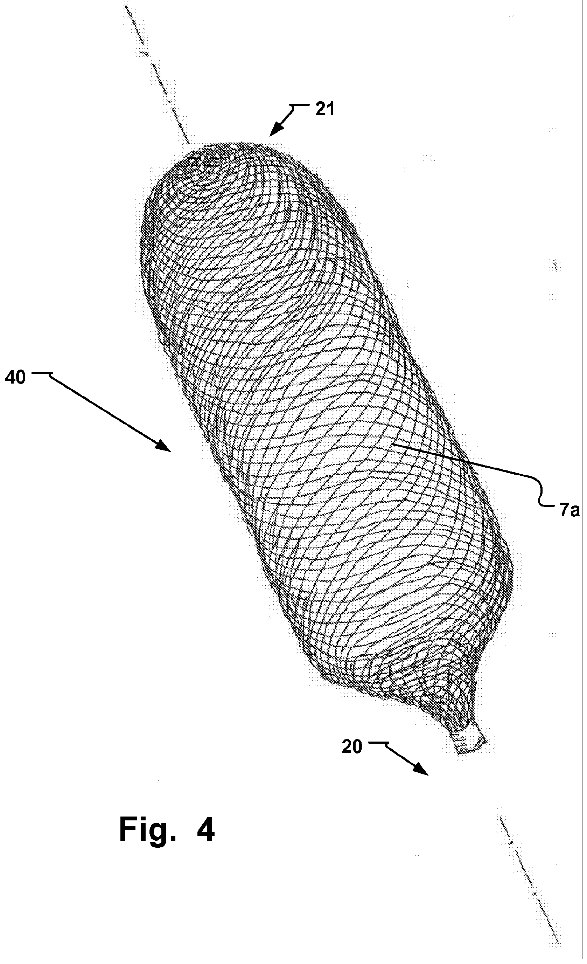

Das nicht erfindungsgemäße Occlusionsinstrument 1 der in Fig. 1 und Fig. 2 dargestellten, Form besteht aus einem Geflecht dünner Drähte oder Fäden, das mit einem Umformungs- und/oder Wärmebehandlungsverfahren eine geeignete Formgebung erhält. Dazu werden nach Fig. 3 und 4 entsprechende Kugel-Drahtgeflechte mit einer proximalen Fassung verwendet.

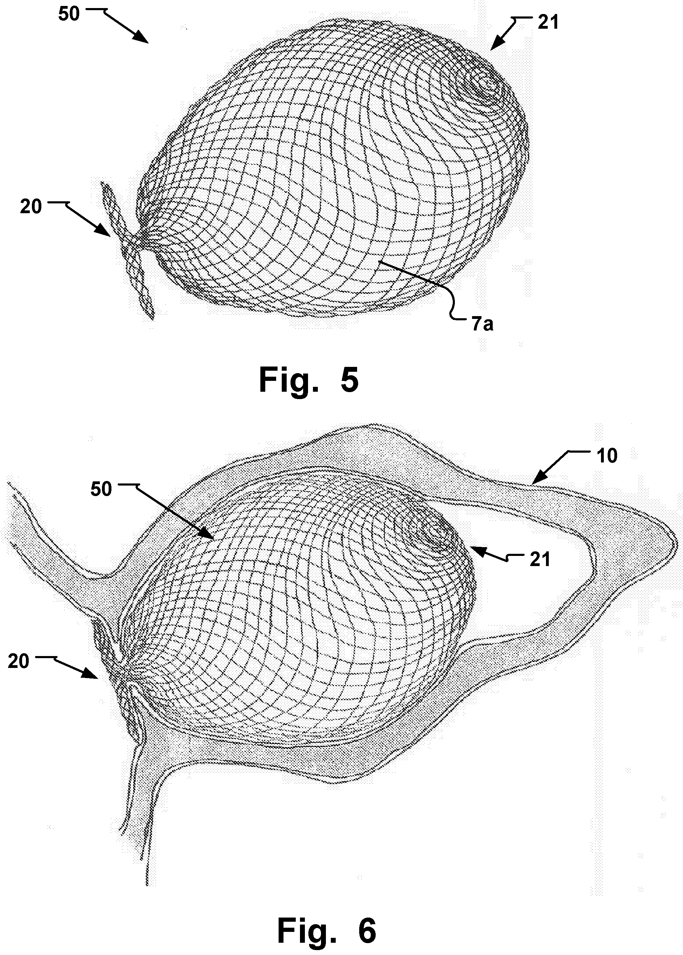

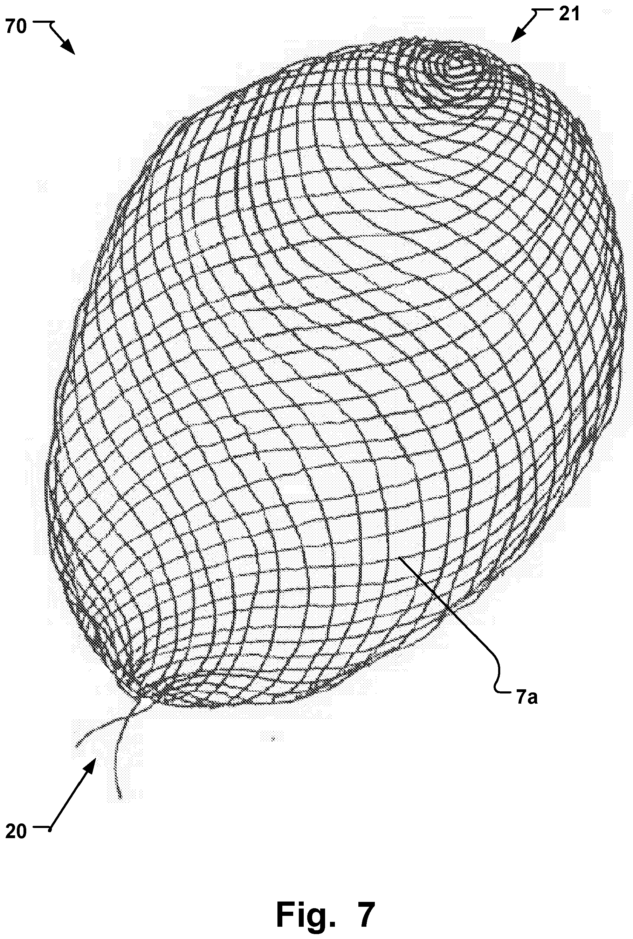

Fig. 5 und 6 zeigen eine Form des erfindungsgemäßen selbst expandierbaren Occlusionsinstruments ohne Fassung bestehend aus einem Geflecht, wie vorstehend beschrieben, aus einem Drahtstück geflochten zu einem Kugelgebinde nach Fig. 7 und mit einem Umformungs- und/oder Wärmebehandlungsverfahren in die gewünschte Endform gebracht.

Bei dem Occlusionsinstrument 1 handelt es sich um einen Herzohroccluder mit einer Formgebung bestehend aus einem vorderseitigen proximalen Retentionsbereich 2, einem Mittenbereich 5 und einem rückseitigen, kugelförmigen distalen Retentionsbereich 3. In dem proximalen Retentionsbereich 2 laufen die Enden der Drähte bzw. Fäden des Geflechts 7 in einer Fassung 4 zusammen. Der distale Retentionsbereich 3 hingegen weist in der bevorzugten Form ein geschlossenes Ende ohne Fassung aus.

Aufgrund der Form ähnlich einer Erdbeere wird das Occlusionsinstrument auch "Strawberry-Occluder" genannt.

Das Geflecht 7 ist aus Drähten oder Fäden gebildet, welche in bevorzugter Weise aus Nitinol oder aus einem anderen Material mit Formgedächtnis oder Memory-Effekt bestehen. Denkbar hierbei wäre es, ein Polymerkunststoff einzusetzen, der die Formgedächtniseigenschaft aufweist. Auch ist es denkbar, ein biologisch abbaubares Formgedächtnismaterial zu verwenden. Wesentlich ist, dass das Geflecht 7 eine ausreichende Flexibilität aufweist, sodass das Occlusionsinstrument 1 auf den Durchmesser eines bei einem minimal-invasiven, insbesondere intravaskulären Operationseingriff verwendeten (nicht explizit dargestellten) Katheters verjüngt werden kann. Aufgrund des Memory-Effekts des Materials verfügt das derart verjüngte Occlusionsinstrument 1 eine Formgedächtnisfunktion, sodass sich das Instrument 1 nach dem Verlassen des Katheters selbstständig expandiert und eine der Verwendung entsprechende, vorbestimmte Form wieder einnimmt. Üblicherweise erfolgt dies, nachdem das zunächst im Katheter angeordnete Occlusionsinstrument 1 an der zu behandelnden Stelle, insbesondere im Herzohr des Herzens eines Patienten, platziert wurde.

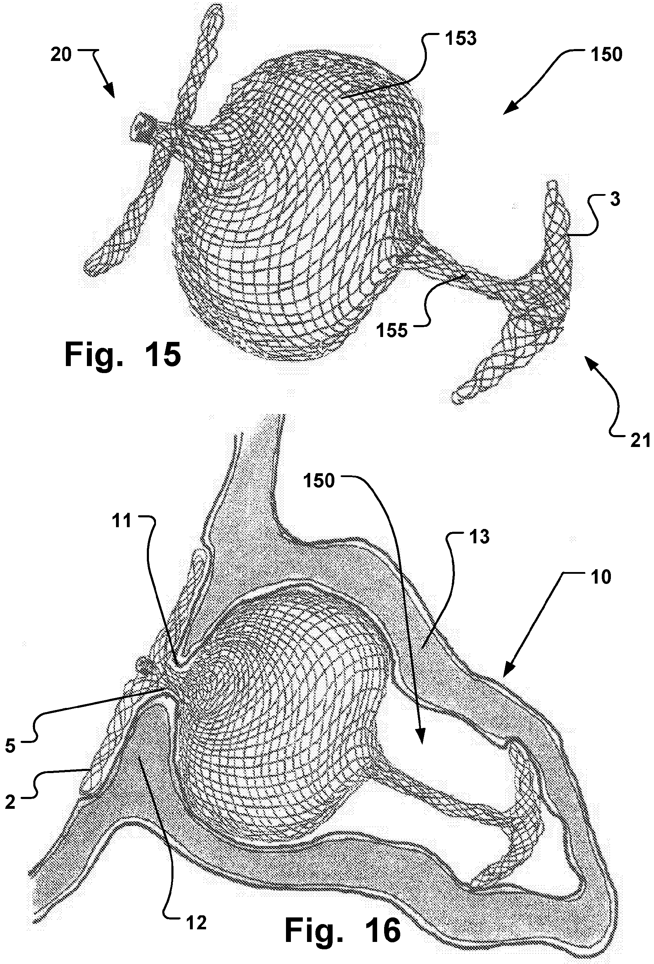

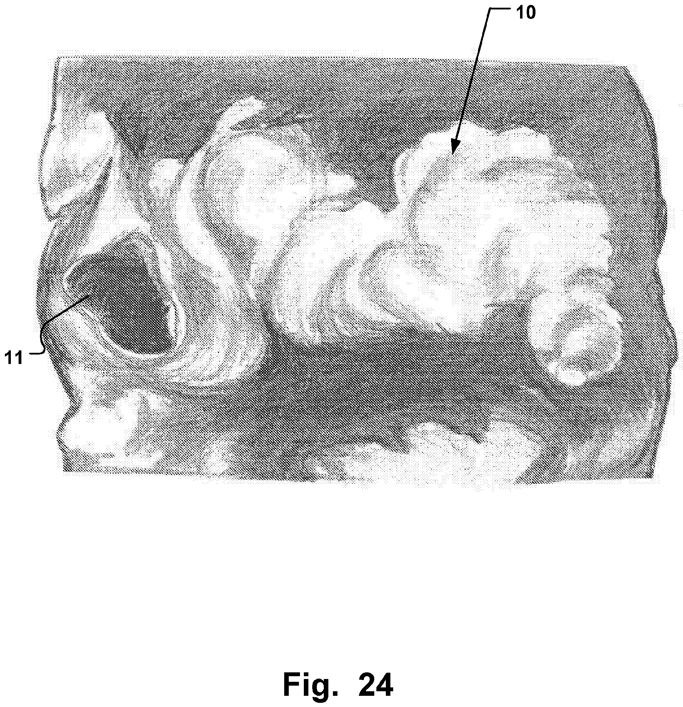

Fig. 15 und 16 stellen einen erfindungsgemäße Variant des Herzohroccluders nach Fig. 1 und 2 dar und können durchaus ihre Anwendung finden auf Grund der hohen Variationsbreite und Spezifik des Herzohres, wie in Fig. 24 dargestellt.

In den Figuren 1, 2, 5 und 6 ist das Occlusionsinstrument in seinem expandierten Zustand dargestellt. Wie bereits angedeutet, weist das Occlusionsinstrument 1 einen proximalen Retentionsbereich 2, einen distalen Retentionsbereich 3 sowie einen taillierten, zylindrischen Mittenbereich 5 auf. Der Herzohroccluder weist ein proximales Ende 20 sowie ein distales Ende 21 auf. Der distale Retentionsbereich 3 mit dem daran ausgebildeten Kugelbereich 6 dient in erster Linie zum Befestigen und Halten des implantierten und expandierten Occlusionsinstruments 1 im Herzohr eines Patienten. Hierzu ist vorgesehen, dass der Kugelbereich 6 in dem zu verschließenden Herzohr an den Innenwandungen des Herzohres zur Anlage kommt und mit den Innenwandungen des Herzohres eine kraftschlüssige Verbindung bildet und somit das implantierte und expandierte Occlusionsinstrument 1 in dem Herzohr hält. Denkbar wäre es beispielsweise, dass der distale Retentionsbereich 3 bzw. der Kugelbereich 6 unter einer radialen Vorspannung stehen, sodass damit für eine relativ breite Variation von Herzohröffnungen der sichere Halt des expandierten Occlusionsinstruments 1 garantiert werden kann.

Im implantierten und expandierten Zustand dient der proximale Retentionsbereich 2 dazu, die Öffnung des Herzohres möglichst optimal zu verschließen. Auf die Einzelheiten der Funktionsweise der einzelnen Retentionsbereiche wird unter Bezugnahme auf die Fig. 2 und 6 im Nachfolgenden näher eingegangen.

Der Konstruktion des Occlusionsinstruments 1 liegt das Prinzip zugrunde, dass sich das Occlusionsinstrument 1 auf die Größe eines Katheters verjüngen lässt. Nach dem Austritt aus dem Katheter entfalten sich dann die Retentionsbereiche 3, 2 selbstständig und legen sich an die Innenwandungen des Herzohres an. Bei der erfindungsgemäßen Konstruktion handelt es sich somit um ein in gewissen Maßen selbstpositionierendes und selbstzentrierendes System. Der Mittenbereich 5 hat dabei eine für die Anwendung vorab festgelegte Länge, um das Verschließen der Herzohröffnung zu gewährleisten.

Durch die flexible Eigenschaft des Occlusionsinstruments 1 aufgrund der verwendeten Materialien und aufgrund des Geflechtes 7 ist das Instrument 1 reversibel zusammen- und auseinanderfaltbar ausgeführt, sodass ein bereits expandiertes Occlusionsinstrument 1 beispielsweise mithilfe eines Explantations- Katheters zusammenfaltbar ist, wobei die kraftschüssige Verbindung zwischen dem Kugelbereich 6 und den Innenwandungen des Herzohres 10 dann gelöst werden.

Fig. 2 und 6 zeigen eine Form des Occlusionsinstruments 1 im implantierten Zustand. Im Einzelnen ist das Occlusionsinstrument im linken Herzohr 10 des Herzens eines Patienten eingesetzt und dient zum Verschließen des Herzohres. Im Einzelnen liegt der Kugelbereich 6 des distalen Retentionsbereiches 3 an den Innenwandungen des Herzohres 10 an und dient zum Positionieren und Fixieren des implantierten Occlusionsinstrumentes 1. Im implantierten Zustand schließt der proximale Retentionsbereich 2 mit der Öffnung des Herzohres 10 ab, wobei die Peripherie des distalen Retentionsbereiches an der Wandung 12 der Herzohröffnung zur Anlage kommt, während der Mittenbereich 5 durch die Öffnung 11 hindurchläuft. Das Occlusionsinstrument 1 stellt von daher ein Verschlusssystem dar, dass mittels minimal-invasiver Verfahren, d.h. beispielsweise über einen Katheter und Führungsdrähte, dem Körper eines Patienten zugeführt und an der dafür bestimmten Stelle positioniert wird. Dabei ist insbesondere der proximale Retentionsbereich 2 des Instruments 1 derart ausgebildet, dass keinerlei Material des implantierten Occlusionsinstruments 1 über die Herzohrwand in den Blutstrom des Patienten gelangen kann. Der Rand des proximalen Retentionsbereiches 2 schließt dabei bündig mit der Herzohrwand 13 ab. Dies erfolgt über einen relativ weiten Bereich unabhängig von dem Herzohrdurchmesser und der Stärke der Herzohrwandung 12 an der Herzohröffnung 11. Dadurch kann erreicht werden, dass nach dem Implantieren des Occlusionsinstruments 1 relativ schnell eine vollständige Endothelialisierung eintritt und mögliche Abwehrreaktionen vom Körper des Patienten nicht eintreten, da ein Blutkontakt mit dem Werkstoff des Implantats 1 wirksam verhindert wird.

Fig. 3 zeigt ein kugelförmiges hohles Kugelgeflecht 30 aus Fäden 7a auf, zur Herstellung eines Herzohroccluders.

Fig. 4 ein ballonförmiges hohles Kugelgeflecht 40 aus Fäden 7a auf, zur Herstellung eines Herzohroccluders.

In Fig. 5 ist in einer Seitenansicht eines Herzohroccluders 50 ohne Fassung gezeigt. Der Herzohroccluder 50 weist am proximalen Ende keine Fassung auf. In Fig. 6 ist die Form des Herzohroccluders 50 nach Fig. 5 im Anschluss an eine Implantation im linken Herzohr 10 des linken Vorhofes gezeigt.

Fig. 7 illustriert ein Kugelgeflecht 70 aus einem Drahtstück ohne Fassung.

Fig. 15 zeigt eine erfindungsgemäße Ausführungsform eines Herzohroccluders 150 mit einer flachen Doppelscheibe am proximalen Ende 20 und einem Zwischen-Retentionsbereich 151. In Fig. 16 wird die Ausführungsform im Anschluss der Implantation in das linke Herzohr 10 gezeigt.

So ist in bevorzugter Weise für das Occlusionsinstrument vorgesehen, dass ein distaler Retentionsbereich mit einer Kugelform derart ausgelegt ist, dass er sich beim Expandieren des Occlusionsinstruments nach außen wölbt, um derart im implantierten Zustand mit den Innenwandungen des Herzohres zur Anlage zu kommen. Mit dieser bevorzugten Ausführungsform ist es demnach möglich, dass das erfindungsgemäße selbst expandierbare Occlusionsinstrument mittels eines Einführ-Kathetersystems besonders tief in das zu verschließende Herzohr vorgeschoben werden kann. Der proximale Retentionsbereich, der in vorteilhafter Weise beispielsweise als proximales Schirmchen ausgebildet ist, wird anschließend, das heißt, nachdem das Occlusionsinstrument mithilfe des Kathetersystems in dem zu verschließenden Herzohr eingeschoben worden ist, zur Entfaltung gebracht und positioniert, wobei das Schirmchen am Rand der Öffnung des Herzohres zum Eingang des Herzohres anliegt. Gleichzeitig expandiert sich auch der distale Bereich des Occlusionsinstruments, das heißt der distale Kugelschirm, wobei beim Expansionsvorgang des distalen Kugelschirmchens der distale Retentionsbereich des Occlusionsinstruments weiter in das Herzohr hineingezogen wird und so über den Mittenbereich eine Zugkraft auf das proximale Schirmchen ausgeübt wird. Als eine direkte Folge hiervon wird das proximale Schirmchen bzw. der proximale Retentionsbereich unter einer permanenten Spannung am Eingang des Herzohres gehalten. Anders ausgedrückt bedeutet dies, dass mit der vorteilhaften Weiterentwicklung des selbst expandierbaren Occlusionsinstruments ein selbstpositionierendes und selbsthaltendes Occlusionsinstrument angegeben wird, wobei die Position des proximalen Schirmchens vorzugsweise bündig an der Öffnung des Herzohres mithilfe des sich selbstständig nach außen wölbenden distalen Kugelschirmchens gehalten wird.

Durch das einerseits flexible und andererseits kraftschlüssige Anliegen des Kugelbereiches mit einer relativ großen Oberfläche an der Innenwandung des Herzohres kann ferner erreicht werden, dass das eingesetzte Occlusionsinstrument deutlich schneller als bei den aus dem Stand der Technik bekannten Verschlusssystemen vollständig von körpereigenem Gewebe eingeschlossen werden kann.

Aus der Verwendung eines aus dünnen Drähten oder Fäden aufgebauten Geflechts als Ausgangsmaterial für das erfindungsgemäße Occlusionsinstrument leitet sich der weitere Vorteil ab, dass es eine langfristige mechanische Stabilität aufweist. Wie bereits angedeutet, kann das Auftreten von Brüchen in der Struktur oder anders artige Materialermüdung des eingesetzten Implantats weitgehend verhindert werden. Ferner besitzt das Geflecht eine ausreichende Steifigkeit.

Dadurch, dass am distalen Retentionsbereich auf eine Fassung zum Zusammenbündeln bzw. Zusammenfassen des Geflechts verzichtet werden kann, ragt auch keine Komponente des Occlusionsinstruments in das Herzohr weiter hinein, sodass das Risiko für Abwehrreaktionen des Körpers oder andere möglichen Komplikationen gering ist.

In einer besonders vorteilhaften Realisierung des erfindungsgemäßen Occlusionsinstruments ist vorgesehen, dass prinzipiell auf jegliche Fassungen verzichtet werden kann, dies betrifft sowohl das proximale als auch distale Ende des Occlussionsinstrumentes. Dabei wurde ein besonderes Kugelgeflecht verwendet, welches aus nur einem Draht hergestellt wird. Grundlage sind die in der Literatur bekannten Verfahren des 2- und 3D-Flechtens, wobei hier die Flechtspindeln auf einer ebenen Fläche schachbrettartig angeordnet sind und untereinander mit beliebigen Richtungsänderungen solche Geflechtsgebilde hergestellt werden können. Eine weitere Herstellungsmöglichkeit von solchen Kugelgeflechten wurde bereits in der Occlutech Patentanmeldung Deutschland Nr. DE102006013770.1 Kugeloccluder beschrieben. Mit dieser Erfindung ist es möglich, einzelne Drahtabschnitte so zu verflechten, dass Kugel-Occluder mit einer Fassung entstehen. DE102006013770.1 ist hiermit in seiner Gesamtheit durch Bezugsnahme beinhaltet.

Reduziert man die Anzahl der Drahtabschnitte auf genau einen Draht mit einer definierten Länge, lassen sich solche Kugelgeflechte ohne Fassungen herstellen. Die beiden übrig gebliebenen Drahtenden werden so miteinander verbunden, beispielsweise verschweißt, dass ein geschlossenes Kugelgeflecht entsteht. Damit kann letztlich bei einem entsprechenden Occlusionsinstrument sowohl distal als auch proximal auf eine Fassung verzichtet werden. Der große Vorteil bei einem solchen Herzohroccluder ist zum Beispiel, dass das proximale Ende des Occluders, welches im linken Herzohr platziert ist, kein Fassungselement trägt, sodass die Bildung von möglichen Tromben extrem reduziert werden kann. Die Drahtenden des einen Drahtes können hierbei an dem proximalen Ende des Herzohroccluders zusammengeführt sein, oder an einer anderen Position.

In den Patentanmeldungen von Occlutech WO 2007 054118, US 2007 112381 und DE 102005053906 sind Vorrichtungen beschrieben, wo am distalen Ende der Vorrichtung zum Implantieren von solchen Occlusionsinstrumenten eine Schlaufe existiert, welche während des Implantationsvorganges die Verbindung zwischen dem Operationsbesteck und dem Occluder herstellt. Von der Dimensionierung können bekannte Einführschleusen mit einem Innendurchmesser von 2-3·3 mn (6 bis 10 French) verwendet werden.

Um zu erreichen, dass das Geflecht des Occlusionsinstruments mittels eines Umformungs- und Wärmebehandlungsverfahrens seine geeignete Formgebung erhalten kann, ist in einer besonders bevorzugten Ausführungsform vorgesehen, dass das Geflecht auf einem Formgedächtnismaterial, insbesondere Nitinol oder Kunststoff gebildet ist. Der Einsatz von Nitinol bei Occlusionsinstrumenten ist bekannt. Formgedächtnispolymere gehören zur Gruppe der intelligenten Polymere und sind Polymere, die einen Formgedächtniseffekt zeigen, d.h. unter Einwirkung eines äußeren Stimulus, wie z.B. einer Temperaturänderung, ihre äußere Form ändern können.

Dabei wird das Polymer zunächst durch konventionelle Verarbeitungsmethoden, wie etwa Spritzguss oder Extrusion, in seine permanente Form gebracht. Anschließend wird der Kunststoff deformiert und in der gewünschten temporären Form fixiert, was auch "Programmierung" genannt wird. Dieser Vorgang kann bei Polymeren einerseits so erfolgen, dass die Probe erwärmt, deformiert und dann abgekühlt wird. Andererseits kann das Polymer bzw. der Kunststoff auch bei niedriger Temperatur deformiert werden, was als "kaltes Verstrecken" bezeichnet wird. Damit ist die permanente Form gespeichert, während die temporäre Form aktuell vorliegt. Wird nun der Polymerformkörper auf eine Temperatur höher als die Schalttemperatur erwärmt, kommt es zum Auslösen des Formgedächtniseffekts und damit zur Wiederherstellung der gespeicherten permanenten Form. Durch Abkühlen der Probe bildet sich die temporäre Form nicht reversibel zurück, weshalb man von einem so genannten Ein-Weg-Formgedächtniseffekt spricht.

Im Vergleich zu bekannten Formgedächtnismaterialien wie z.B. der Formgedächtnislegierung Nitinol, einer äquiatomaren Legierung aus Nickel und Titan, sind Formgedächtnispolymere mit ihren Gedächtnisleistungen um ein vielfaches Überlegen. Dabei ist nur ein geringer Aufwand (Erwärmen bzw. Abkühlen) zur Programmierung der temporären Form bzw. zur Wiederherstellung der permanenten Form nötig. Darüber hinaus beträgt bei Nitinol die maximale Deformation zwischen permanenter und temporärer Form nur 8%. Formgedächtnispolymere weisen wesentlich höhere Verformbarkeit von bis zu 1.100% auf. Sämtliche vorgenannten Formgedächtnispolymere und Materialien werden mit der vorliegenden Erfindung für die biomedizinische Anwendung des erfindungsgemäßen Occlusionsinstruments beansprucht.

In einer vorteilhaften Weiterentwicklung der zuletzt genannten Ausführungsform des erfindungsgemäßen Occlusionsinstruments, bei welchem das Geflecht aus einem Formgedächtnismaterial gebildet wird, ist vorgesehen, dass das Material ein biologisch abbaubares Formgedächtnis-Polymermaterial aufweist. Insbesondere eignen sich synthetische, bioabbaubare Implantatmaterialien. Derartige abbaubare Werkstoffe bzw. Polymere enthalten unter physiologischen Bedingungen spaltbare Bindungen. Dabei spricht man von einer "Bioabbaubarkeit", wenn der Werkstoff unter Verlust der mechanischen Eigenschaft durch oder in einem biologischen System abgebaut wird. Die äußere Form und die Masse des Implantats bleibt während des Abbaus unter Umständen erhalten. Wird von einer Degradationszeit ohne zusätzliche quantifizierende Angaben gesprochen, so ist die Zeit, in der der vollständige Verlust der mechanischen Eigenschaft auftritt, gemeint. Unter biostabilen Werkstoffen versteht man solche, die in biologischen Systemen stabil sind und langfristig zumindest nur teilweise abgebaut werden.

Bei abbaubaren Polymeren unterscheidet man zwischen hydrolytisch und enzymatisch abbaubare Polymere. Der hydrolytische Abbau hat den Vorteil, dass die Abbaugeschwindigkeit unabhängig vom Ort der Implantation ist, da Wasser überall vorhanden ist. Demgegenüber ist die Konzentration an Enzymen lokal sehr unterschiedlich. Bei bioabbaubaren Polymeren oder Werkstoffen kann demnach der Abbau durch reine Hydrolyse, enzymatisch induzierte Reaktionen oder durch deren Kombination erfolgen. Typische hydrolysierbare chemische Bindungen sind Amid-, Ester- oder Acetal-Bindungen. Beim Abbau beobachtet man zwei Mechanismen. Beim Oberflächenabbau findet die Hydrolyse chemischer Bindungen ausschließlich an der Oberfläche statt. Aufgrund des hydrophoben Charakters erfolgt der Polymerbau schneller als die Diffusion von Wasser in das Innere des Materials. Diesen Mechanismus beobachtet man vor allem bei Poly(anhydride)n oder Poly(orthoester)n. Für die vor allem für den Formgedächtniseffekt bedeutsamen Poly(hydroxcarbonsäuren), wie Poly (milchsäure) oder Poly(glycosesäure) bzw. entsprechende Copolymere, erfolgt der Polymerabbau im gesamten Volumen. Der geschwindigkeitsbestimmende Schritt ist hierbei die hydrolytische Bindungsspaltung, da die Diffusion von Wasser in der eher hydrophylen Polymermatrix relativ schnell erfolgt. Für die Anwendung von bioabbaubaren Polymeren ist entscheidend, dass sie einerseits mit einer kontrollier- bzw. einstellbaren Geschwindigkeit abbauen und andererseits die Abbauprodukte nicht toxisch sind.

Sämtliche vorgenannten biologisch abbaubaren Formgedächtnis-Polymere werden erfindungsgemäß einschließlich der weiteren möglichen Anwendungen aus dem Patent Deutschland-Anmeldung "medizinisches selbstexpandierendes Occlusionsinstrument"DE 10 2005 053 958, WO 2007 054117 und US 2007167980 mit ihrer Anwendung auf die in diesem Patent beschriebene bevorzugte Herzoccludervariante einschließlich der dargestellten, abgeleiteten Varianten, beansprucht.

Gemäß Ausführungsformen des Occlusionsinstrumentes bestehen die Fäden 7a des Geflechts 7 aus einer Formgedächtnis-Polymerkomposition, sodass sich das Geflecht 7 unter Einwirken eines äußeren Stimulus von einer temporären Form zu einer permanenten Form verformt, wobei die temporäre Form in der ersten Formgebung des Geflechts 7 und die permanente Form in der zweiten Formgebung des Geflechts 7 vorliegt. Der äußere Stimulus kann eine festlegbare Schalttemperatur sein, beispielsweise im Bereich zwischen der Raumtemperatur und der Körpertemperatur eines Patienten.

Die Polymerkomposition gewisser Ausführungsformen weist polymere Schaltelemente auf, wobei unterhalb der festlegbaren Schalttemperatur die temporäre Form des Geflechts 7 mithilfe von charakteristischen Phasenübergängen der polymeren Schaltelemente stabilisiert wird.

Gemäß bestimmter Ausführungsformen weist die Polymerkomposition ein kristallines oder teilkristallines Polymernetzwerk mit kristallinen Schaltsegmenten auf, wobei die temporäre Form des Geflechts 7 durch ein Einfrieren der kristallinen Schaltsegmente beim Kristallisationsübergang fixiert und stabilisiert wird, wobei die Schalttemperatur durch die Kristallisationstemperatur bzw. Schalttemperatur der kristallinen Schaltsegmente bestimmt wird.

Gemäß bestimmter Ausführungsformen weist die Polymerkomposition ein amorphes Polymernetzwerk mit amorphen Schaltsegmenten auf, wobei die temporäre Form des Geflechts 7 durch ein Einfrieren der amorphen Schaltsegmente beim Glasübergang der Schaltsegmente fixiert und stabilisiert wird, wobei die Schalttemperatur durch die Glasübergangstemperatur der amorphen Schaltsegmente bestimmt wird.

Gemäß bestimmter Ausführungsformen weist die Polymerkomposition ein lineares, phasensegregiertes Multiblock-Copolymernetzwerk auf, welches in zumindest zwei verschiedenen Phasen vorliegen kann, wobei die erste Phase eine Hartsegment-bildende Phase ist, bei welcher im Polymer eine Vielzahl von Hartsegment-bildende Blöcke ausgebildet werden, welche zur physikalischen Vernetzung der Polymerstruktur dienen und die permanente Form des Geflechts 7 bestimmen und stabilisieren, und wobei die zweite Phase eine Schaltsegment-bildende Phase ist, bei welcher im Polymer eine Vielzahl von Schaltsegment-bildende Blöcke ausgebildet werden, die zur Fixierung der temporären Form des Geflechts 7 dienen, wobei die Übergangstemperatur der Schaltsegment-bildenden Phase zur Hartsegment-bildenden Phase die Schalttemperatur ist, und wobei oberhalb der Übergangstemperatur der Hartsegment-bildenden Phase die Formgebung des Geflechts 7 durch konventionelle Verfahren, insbesondere durch Spritzguss- oder Extrusionsverfahren, eingestellt werden kann.

Die Polymerkomposition kann thermoplastische Polyurethan-Elastomere mit einer Multiblockstruktur aufweisen, wobei die Hartsegment-bildende Phase durch eine Umsetzung von Diisocyanaten, insbesondere Methylen-bis(4-phenylisocyanat) oder Hexamethylendiisocyanat, mit Diolen, insbesondere 1,4-Butandiol, gebildet wird, und wobei sich die Schaltsegment-bildende Phase aus oligomeren Polyether-bzw. Polyesterdiolen, insbesondere ausgehend von OH-terminierten Poly(tetrahydrofuran), Poly(ε-caprolaceton), Poly(ethylenadipat), Poly(ethylenglyocol) oder Poly(propylenglycol), ergibt.

Die Polymerkomposition kann auch phasensegregierte Diblockcopolymere mit einem amorphen A-Block und einem teilkristallinen B-Block aufweisen, wobei der Glasübergang vom amorphen A-Block die Hartsegment-bildende Phase bildet, und wobei die Schmelztemperatur des teilkristallinen B-Blocks als Schalttemperatur für den thermischen Formgedächtnis-Effekt dient. Die Polymerkomposition kann Polystyrol als amorphen A-Block und Poly(1,4-butadien) als teilkristallinen B-Block aufweisen.

Gemäß bestimmter Ausführungsformen weist die Polymerkomposition ein phasensegregiertes Triblockcopolymer mit einem teilkristallinen zentralen B-Block und mit zwei amorphen terminalen A-Blöcken auf, wobei die A-Blöcke das Hartsegment aufbauen, und wobei der B-Block die Schalttemperatur bestimmt. Die Polymerkomposition kann beispielsweise teilkristallines Poly(tetrahydrofuran) als zentralen B-Block und amorphes Poly(2-methyloxazolin) als terminale A-Blöcke aufweisen.

Gemäß bestimmter Ausführungsformen weist die Polymerkomposition Polynorbornen, Polyethylen-Nylon-6-Pfropfcopolymere und/oder vernetzte Poly (ethylen-co-vinylacetat)-Copolymere aufweist.

Gemäß bestimmter Ausführungsformen weist die Polymerkomposition ein kovalentes vernetztes Polymernetzwerk auf, welches durch Polymerisation, Polykondensation und/oder Polyaddition von difunktionellen Monomeren oder Makromeren unter Zusatz von tri- oder höherfunktionellen Vernetzern aufgebaut ist, wobei über eine geeignete Auswahl der Monomere, deren Funktionalität und dem Anteil am Vernetzer die chemischen, thermischen und mechanischen Eigenschaften des gebildeten Polymernetzwerkes gezielt einstellbar sind. Die Polymerkomposition kann hierbei beispielsweise ein kovalentes Polymernetzwerk sein, welches durch vernetzende Copolymerisation von Stearylacrylat und Methacrylsäure mit N,N'-Methylenbisacrylamid als Vernetzer aufgebaut ist, wobei der Formgedächtnis-Effekt der Polymerkomposition auf kristallisierenden Stearylseitenketten beruht.

Gemäß bestimmter Ausführungsformen weist die Polymerkomposition ein kovalent vernetztes Polymernetzwerk auf, welches durch eine nachträgliche Vernetzung von linearen oder verzweigten Polymeren gebildet wird. Die Vernetzung kann durch ionisierende Strahlung oder durch thermische Spaltung radikal bildender Gruppe ausgelöst werden.

Gemäß bestimmter Ausführungsformen weist die Polymerkomposition zumindest ein biologisch abbaubares Material auf. Die Polymerkomposition kann hierbei beispielsweise ein hydrolytisch abbaubares Polymer aufweisen, insbesondere Poly(hydroxycarbonsäuren) bzw. entsprechende Copolymere. Die Polymerkomposition weist besipielsweise enzymatisch abbaubare Polymere auf. Gemäß bestimmter Ausführungsformen weist die Polymerkomposition ein bioabbaubares thermoplastisches amorphes Polyurethan-Copolyester-Polymernetzwerk auf. Die Polymerkomposition kann ein bioabbaubares elastisches Polymernetzwerk aufweisen, welches durch Vernetzung von oligomeren Diolen mit Diisocyanat erhalten wird. Die Polymerkomposition kann hierbei auf der Basis von kovalenten Netzwerken ausgehend von Oligo(ε-caprolacton)dimethacrylat und Butylacrylat gebildet sein.

Besonders bevorzugt ist vorgesehen, dass sich das Geflecht des erfindungsgemäßen Occlusionsinstruments auf den Durchmesser eines bei dem minimal-invasiven Operationseingriff verwendeten Katheters verjüngen lässt. Der Vorteil dieser Ausführungsform ist darin zu sehen, dass die zur Implantation und Explantation zu verwendenden Kathetersysteme einen deutlich reduzierten Innendurchmesser aufweisen können, was die Manövrierbarkeit des zu implantierenden Occlusionsinstruments deutlich erhöht. Von daher kann die Positioniergenauigkeit des Instruments im Herzohr verbessert werden. Bei einem aus Nitinol bestehenden Occluder liegt der Innendurchmesser des zur Implantation oder Explantation verwendeten Katheters zwischen 8 bis 10 French, wohingegen bei der Verwendung von Occlusionsinstrumenten aus Polymerkunststoff der Innendurchmesser lediglich zwischen 6 bis 8 French liegen muss.

Schließlich ist besonders bevorzugt vorgesehen, dass das Occlusionsinstrument wenigstens eine Gewebeeinlage 120, 130 aufweist, die zum vollständigen Verschließen des Herzohrs im bzw. am proximalen Retentionsbereich angeordnet ist. Diese Gewebeeinlage dient dazu, die in den sich erweiternden Durchmessern des Occlusionsinstruments verbleibenden Zwischenräume nach dem Einsetzen und Expandieren des Instruments im Herzohr zu verschließen. Die Gewebeeinlage wird beispielsweise am distalen Retentionsbereich am Geflecht des Occlusionsinstruments derart befestigt, dass sie wie ein Tuch über den distalen Retentionsbereich gespannt werden kann. Der Vorteil dieser Konstruktion liegt darin, dass sich der Randsaum des distalen Retentionsbereiches bündig an die Herzohröffnung anlegt und weniger Fremdmaterial in den Körper des Patienten eingebracht wird. Die Gewebeeinlagen können beispielsweise aus Dacron (Polyethylenterephthalat) hergestellt sein. Selbstverständlich sind hier aber auch andere Materialien und andere Positionen der Gewebeeinlage im bzw. am Occlusionsinstrument denkbar einschließlich biologisch abbaubarer Materialien, welche vorstehend beschrieben wurden.

Die erfindungsgemäße Lösung weist eine Reihe wesentlicher Vorteile gegenüber der aus dem Stand der Technik bekannten und vorstehend genannten Occlusionsinstrumenten auf. Zum einen handelt es sich bei dem erfindungsgemäßen Occluder um ein selbst expandierbares Instrument, was auf besonders einfache Weise mit beispielsweise einem geeigneten Einführkatheter implantierbar ist. Hierzu wird beispielsweise eine Vene im Bereich der Leiste des Patienten punktiert und das Einführkathetersystem bis an die Scheidewand des rechten Vorhofs vorgeführt. Mittels einer Punktion der Scheidewand des Vorhofs, was beispielsweise eine bekannte transseptale Punktion sein kann, wird der linke Vorhof des Herzens erreicht, sodass anschließend von der Leistenvene aus das Einführkathetersystem in das linke Herzohr eingebracht werden kann. Über das Einführkathetersystem kann anschließend das selbst expandierbare Occlusionsinstrument zum Verschließen des Herzohres eingebracht werden, die entsprechenden Schleusen haben einen verhältnismäßig kleinen Querschnitt, maximal 9 bis 10 F.

Das Occlusionsinstrument, welches während der Implantation im zusammengefalteten Zustand vorliegt, weist in bevorzugter Weise einen Durchmesser von 6 bis 10 French auf, sodass der Eingriff zum Verschließen des Herzohres minimal-invasiv ist.

Nachdem das zusammengefaltete Occlusionsinstrument mithilfe beispielsweise des Einführkatheters in dem zu verschließenden Herzohr positioniert ist, wird das Occlusionsinstrument von dem Katheter freigegeben, worauf sich dieses infolge seiner selbst expandierbaren Natur auseinander faltet und die mittels des bei Herstellung angewandten Umformungs- und Wärmebehandlungsverfahrens ausgeprägte Formgebung einnimmt. In diesem expandierten Zustand ist der rückseitige distale Retentionsbereich mit dem daran ausgebildeten Kugelbereich vollständig auseinander gefaltet und kommt an den Innenwandungen des zu verschließenden Herzohres zur Anlage. Dabei dient der distale Retentionsbereich mit dem daran ausgebildeten Kugelbereich zur Befestigung und Positionierung des expandierten Occlusionsinstruments in dem Herzohr. Der sich von dem distalen Retentionsbereich in Richtung Herzohröffnung erstreckende Mittenbereich sowie der am proximalen Ende des Mittenbereiches vorgesehene proximale Retentionsbereich füllen dabei den Öffnungsbereich des Herzohres nahezu vollständig aus, sodass das gesamte expandierte Occlusionsinstrument im eingesetzten Zustand als ein Verschlussstöpsel zum Verschließen des Herzohres dient. Auf diese Weise kann in besonders einfacher und minimal-invasiver Weise eine Trombusbildung mit dem Risiko eines Schlaganfalls erheblich reduziert werden.

Insbesondere dadurch, dass die Positionierung und Fixierung des Occlusionsinstruments mithilfe des an den Innenwandungen des Herzohres zur Anlage kommenden kugelförmigen Gebildes erreicht wird, kann bei dem Occlusionsinstrument auf Befestigungshäkchen oder andere Verankerungsmittel verzichtet werden, die üblicherweise bei derartigen Occlusionsinstrumenten zur Fixierung und Positionierung des Instrumentes im Gewebe verwendet werden. Dabei ist insbesondere zu beachten, dass infolge der äußerst dünnwandigen Ausgestaltung des Gewebes in der Umgebung des Herzohres die üblicherweise eingesetzten Befestigungshäkchen keine dauerhafte Befestigung und Positionierung des Occlusionsinstrumentes bereitstellen können. Mit der erfindungsgemäßen Lösung und insbesondere durch den am distalen Retentionsbereich ausgebildeten Kugelbereich kann die Problematik der Befestigung des Occlusionsinstruments an dem äußerst dünnwandigen und leicht zu verletzenden Herzohrgewebe mittels Häkchen umgangen werden.

Mit dem erfindungsgemäßen Verfahren wird eine besonders leicht zu realisierende Möglichkeit zur Herstellung des vorstehend beschriebenen Occlusionsinstruments angegeben. Dabei wird zunächst ein kugelförmiges Hohlgeflecht mittels beispielsweise einer Rundflechtmaschine ausgebildet. Es kann hier eine Technik zum Einsatz kommen, bei der das ausgebildete Geflecht am Ende der Flechtlänge, d.h. am späteren distalen Ende des Occlusionsinstruments, gebündelt wird und am Anfang der Flechtlänge, d.h. am späteren distalen Ende des Occlusionsinstruments, geschlossen bleibt. Damit ist es möglich, ein kugelförmiges Hohlgeflecht herzustellen, dessen gebündeltes Ende dem proximalen Ende des fertigen Occlusionsinstruments und dessen gegenüberliegendes geschlossene Ende dem distalen Ende des fertigen Occlusionsinstruments entspricht. Dadurch, dass zum Herstellen des Occlusionsinstruments ein an sich bekanntes Flechtverfahren verwendet werden kann, weist das gefertigte Occlusionsinstrument mechanische Eigenschaften hinsichtlich beispielsweise der Dehnung, Stabilität, Festigkeit etc. auf, die individuell an den späteren Einsatz des Occlusionsinstruments angepasst werden können. In vorteilhafter Weise können metallische Drähte, aber auch organische Fäden zu dem Geflecht verarbeitet werden.

Ebenso können Kugelgeflechte, wie beschrieben ohne Fassung zum Einsatz kommen.

Hinsichtlich des Verfahrens ist in bevorzugter Weise vorgesehen, dass der Verfahrensschritt des Ausformens der Retentionsbereiche und des Mittenbereichs einen Umformungs- und/oder Wärmebehandlungsschritt aufweist. Dieses ist insbesondere dann von Vorteil, wenn das ausgebildete kugelförmige Hohlgeflecht aus Nitinol oder aus einem anderen Material, insbesondere Polymer, mit Formgedächtnis- oder Memory-Effekt besteht. Für das erfindungsgemäße Occlusionsinstrument ist insbesondere vorgesehen, dass das Geflecht aus einem Formgedächtnispolymer ausgebildet ist, welches beispielsweise auf Polyanhydriden als Matrix oder auf Polyhydroxycarbonsäure basiert. Hierbei handelt es sich um synthetische abbaubare Materialien, die über einen thermisch induzierten Formgedächtniseffekt verfügen. Denkbar wären aber auch andere Formgedächtnis-polymere, wie etwa Blockcopolymere wie sie beispielsweise im Sonderdruck angewandte Chemie 2002, 114, Seiten 2138 bis 2162 von A. Lendlein und S. Kelch beschrieben werden. Derartige Materialien lassen sich auf einfache Weise durch eine Kombination aus Umformungs- und Wärmebehandlungsschritten in eine entsprechende endgültige Form bringen. Ein fertig ausgebildeter Occluder lässt sich dann beispielsweise auf die Größe eines Katheters verjüngen. Nach dem Austritt aus dem Katheter entfaltet sich das Occlusionsinstrument dann selbstständig und nimmt wieder jene Formgebung an, die mittels des Umformungs- und/oder Wärmebehandlungsschritts dem kugel- bzw. trichterförmigen Hohlgeflecht des Occlusionsinstruments beim Herstellungsverfahren ausgeprägt wurde.

In bevorzugter Weise ist das kugelförmige Hohlgeflecht derart hergestellt, dass dünne Drähte oder Fäden, das fertige Geflecht begründen, beim Ausbilden des kugelförmigen Hohlgeflechts am distalen Ende des Geflechts untereinander verflochten werden. Dies stellt eine mögliche und besonders einfach zu realisierende Art und Weise dar, ein Occlusionsinstrument gemäß der vorliegenden Erfindung herzustellen, dessen distaler Retentionsbereich eine zum distalen Ende hin geschlossene Form aufweist. Selbstverständlich sind aber auch andere Herstellungsverfahren denkbar.

Zur Fertigung der Drahtgeflechte 7 wird beispielsweise auf das bereits erteilte Europapatent EP1651117B1 und die Occlutech-Patentanmeldung DE 102006013770 Kugeloccluder verwiesen.

Es sei darauf hingewiesen, dass die Ausführung der Erfindung nicht auf das in den Figuren beschriebene Ausführungsbeispiel beschränkt ist, sondern auch in einer Vielzahl von Varianten möglich ist.

### Bezugszeichenliste

- 1: Occlusionsinstrument
- 2: proximaler Retentionsbereich
- 3: distaler Retentionsbereich
- 4: Fassung
- 5: Mittenbereich
- 6: Kugelbereich
- 7: Geflecht
- 7a: Faden des Geflechts 7
- 8: Vertiefung
- 9: Verbindungselement
- 10: Herzohr
- 11: Herzohröffnung
- 12: Wandung der Herzohröffnung
- 13: Herzohrwand
- 20: proximales Ende
- 21: distales Ende
- 30: kugelförmiges hohles Kugelgeflecht
- 40: ballonförmiges hohles Kugelgeflecht
- 50: Herzohroccluder ohne Fassung
- 70: Kugelgeflecht aus einem Drahtstück ohne Fassung
- 150: Erfindungsgemäßer Herzoccluder
- 153: Zwischen-Retentionsbereich
- 155: Hinterer Mittenbereich

## Patentansprüche

1. Occlusionsinstrument (150) bestehend aus einem Geflecht (7) aus mindestens einem Draht oder Faden (7a), wobei das Occlusionsinstrument (150) mit einem Umformungs- und/oder Wärmebehandlungsverfahren eine geeignete Formgebung erhalten hat, selbst expandierbar ist, und zur sicheren Verankerung in einem Herzohr (10) des linken oder rechten Vorhofes eines Herzens konfiguriert ist, umfassend einen scheibenförmigen proximalen Retentionsbereich (2) an einem proximalen Ende (20) des Occlusionsinstrumentes (150) zum Verschließen einer Öffnung des Herzohres, und
wobei das Occlusionsinstrument (150) ein geschlossenes distales Ende (21) ohne Fassung aufweist,
**gekennzeichnet durch** einen Zwischen-Retentionsbereich (153) des Occlusionsinstrumentes (150), welcher im wesentlich kugelförmig, hohl ausgeformt ist, einen distalen Retentionsbereich (3), einen Zwischen-Retentionsbereich (153), sowie einen distalen Mittenbereich (155) angeordnet zwischen dem distalen Retentionsbereich (3) und dem Zwischen-Retentionsbereich (153), und einen proximalen Mittenbereich (5) zwischen dem proximalen Retentionsbereich (2) und dem genannten Zwischen-Retentionsbereich (153); wobei
der distale Mittenbereich (155) des Occlusionsinstrumentes (150) zylindrisch länglich ist und Flexibilität des distalen Retentionsbereiches (3) zum Zwischen-Retentionsbereich (153) bereitstellt.

2. Occlusionsinstrument (150) gemäß Anspruch 1, wobei der mindestens eine Draht oder Faden in dem proximalen Retentionsbereich (2) in einer Fassung zusammenläuft.

3. Occlusionsinstrument (150) gemäß Anspruch 1 oder 2, wobei das Occlusionsinstrument (150) im genannten Herzohr des linken oder rechten Vorhofes selbstpositionierend und selbstzentrierend ist.

4. Occlusionsinstrument (150) gemäß Anspruch 1, wobei der proximale Retentionsbereich (2) eine Vertiefung (8) aufweist, in der eine Fassung (4) angeordnet ist, in welcher die Enden der Drähte bzw. Fäden des Geflechts (7) zusammenlaufen.

5. Occlusionsinstrument (150) gemäß einem der vorstehenden Ansprüche, wobei das Occlusionsinstrument in zusammengefalteten Zustand einen Durchmesser von 2-3.3 mm (6-10 French) aufweist, sodass der Eingriff zum Verschließen des Herzohres minimal-invasiv ist.

6. Occlusionsinstrument (150) gemäß einem der vorstehenden Ansprüche, wobei das Geflecht (7) ein Kugel-Drahtgeflecht ist.

7. Occlusionsinstrument (150) gemäß Anspruch 6, wobei das Kugel-Drahtgeflecht aus mehreren Drähten besteht und eine proximale Fassung die Enden der Drähte zusammenhält.

8. Occlusionsinstrument (150) gemäß Anspruch 6, wobei das Kugel-Drahtgeflecht aus einem Draht besteht und die Enden des Drahtes ohne Hülse miteinander verbunden sind.

9. Occlusionsinstrument gemäß Ansprüchen 1 bis 8, wobei die Fäden des Geflechts (7) aus einer Formgedächtnis-Polymerkomposition bestehen, sodass sich das Geflecht (7) unter Einwirken eines äußeren Stimulus von einer temporären Form zu einer permanenten Form verformt, wobei die temporäre Form in der ersten Formgebung des Geflechts (7) und die permanente Form in der zweiten Formgebung des Geflechts (7) vorliegt.

10. Occlusionsinstrument gemäß Anspruch 9, wobei der äußere Stimulus eine festlegbare Schalttemperatur ist, wobei die Schalttemperatur im Bereich zwischen der Raumtemperatur und der Körpertemperatur des Patienten liegt.

11. Occlusionsinstrument gemäß Anspruch 10, wobei die Polymerkomposition polymere Schaltelemente aufweist, wobei unterhalb der festlegbaren Schalttemperatur die temporäre Form des Geflechts (7) mithilfe von charakteristischen Phasenübergängen der polymeren Schaltelemente stabilisiert wird.

12. Occlusionsinstrument gemäß Anspruch 11, wobei die Polymerkomposition ein kristallines oder teilkristallines Polymernetzwerk mit kristallinen Schaltsegmenten aufweist, wobei die temporäre Form des Geflechts (7) durch ein Einfrieren der kristallinen Schaltsegmente beim Kristallisationsübergang fixiert und stabilisiert wird, wobei die Schalttemperatur durch die Kristallisationstemperatur bzw. Schalttemperatur der kristallinen Schaltsegmente bestimmt wird.

13. Occlusionsinstrument gemäß Anspruch 11, wobei die Polymerkomposition ein amorphes Polymernetzwerk mit amorphen Schaltsegmenten aufweist, wobei die temporäre Form des Geflechts (7) durch ein Einfrieren der amorphen Schaltsegmente beim Glasübergang der Schaltsegmente fixiert und stabilisiert wird, wobei die Schalttemperatur durch die Glasübergangstemperatur der amorphen Schaltsegmente bestimmt wird.

14. Occlusionsinstrument gemäß einem der vorhergehenden Ansprüche 9-13, wobei die Polymerkomposition ein lineares, phasensegregiertes Multiblock-Copolymernetzwerk aufweist, welches in zumindest zwei verschiedenen Phasen vorliegen kann, wobei die erste Phase eine Hartsegment-bildende Phase ist, bei welcher im Polymer eine Vielzahl von Hartsegment-bildende Blöcke ausgebildet werden, welche zur physikalischen Vernetzung der Polymerstruktur dienen und die permanente Form des Geflechts (7) bestimmen und stabilisieren, und wobei die zweite Phase eine Schaltsegment-bildende Phase ist, bei welcher im Polymer eine Vielzahl von Schaltsegment-bildende Blöcke ausgebildet werden, die zur Fixierung der temporären Form des Geflechts (7) dienen, wobei die Übergangstemperatur der Schaltsegment-bildenden Phase zur Hartsegment-bildenden Phase die Schalttemperatur ist, und wobei oberhalb der Übergangstemperatur der Hartsegment-bildenden Phase die Formgebung des Geflechts (7) durch konventionelle Verfahren, insbesondere durch Spritzguss- oder Extrusionsverfahren, eingestellt werden kann.

15. Occlusionsinstrument gemäß Anspruch 14, wobei die Polymerkomposition thermoplastische Polyurethan-Elastomere mit einer Multiblockstruktur aufweist, wobei die Hartsegment-bildende Phase durch eine Umsetzung von Diisocyanaten, insbesondere Methylen-bis(4-phenylisocyanat) oder Hexamethylendiisocyanat, mit Diolen, insbesondere 1,4-Butandiol, gebildet wird, und wobei sich die Schaltsegment-bildende Phase aus oligomeren Polyether-bzw. Polyesterdiolen, insbesondere ausgehend von OH-terminierten Poly(tetrahydrofuran), Poly(ε-caprolaceton), Poly(ethylenadipat), Poly (ethylenglyocol) oder Poly(propylenglycol), ergibt.

16. Occlusionsinstrument gemäß Anspruch 14, wobei die Polymerkomposition phasensegregierte Diblockcopolymere mit einem amorphen A-Block und einem teilkristallinen B-Block aufweist, wobei der Glasübergang vom amorphen A-Block die Hartsegment-bildende Phase bildet, und wobei die Schmelztemperatur des teilkristallinen B-Blocks als Schalttemperatur für den thermischen Formgedächtnis-Effekt dient.

17. Occlusionsinstrument gemäß Anspruch 16, wobei die Polymerkomposition Polystyrol als amorphen A-Block und Poly(1,4-butadien) als teilkristallinen B-Block aufweist.

18. Occlusionsinstrument gemäß Anspruch 14, wobei die Polymerkomposition ein phasensegregiertes Triblockcopolymer mit einem teilkristallinen zentralen B-Block und mit zwei amorphen terminalen A-Blöcken aufweist, wobei die A-Blöcke das Hartsegment aufbauen, und wobei der B-Block die Schalttemperatur bestimmt.

19. Occlusionsinstrument nach Anspruch 18, wobei die Polymerkomposition teilkristallines Poly(tetrahydrofuran) als zentralen B-Block und amorphes Poly(2-methyloxazolin) als terminale A-Blöcke aufweist.

20. Occlusionsinstrument gemäß einem der vorhergehenden Ansprüche 9-19, wobei die Polymerkomposition Polynorbornen, Polyethylen-Nylon-6-Pfropfcopolymere und/oder vernetzte Poly(ethylen-co-vinylacetat)-Copolymere aufweist.

21. Occlusionsinstrument gemäß einem der vorhergehenden Ansprüche 9-20, wobei die Polymerkomposition ein kovalentes vernetztes Polymernetzwerk aufweist, welches durch Polymerisation, Polykondensation und/oder Polyaddition von difunktionellen Monomeren oder Makromeren unter Zusatz von tri- oder höherfunktionellen Vernetzern aufgebaut ist, wobei über eine geeignete Auswahl der Monomere, deren Funktionalität und dem Anteil am Vernetzer die chemischen, thermischen und mechanischen Eigenschaften des gebildeten Polymernetzwerkes gezielt einstellbar sind.

22. Occlusionsinstrument gemäß Anspruch 21, wobei die Polymerkomposition ein kovalentes Polymernetzwerk ist, welches durch vernetzende Copolymerisation von Stearylacrylat und Methacrylsäure mit N,N'-Methylenbisacrylamid als Vernetzer aufgebaut ist, wobei der Formgedächtnis-Effekt der Polymerkomposition auf kristallisierenden Stearylseitenketten beruht.

23. Occlusionsinstrument gemäß einem der vorhergehenden Ansprüche 9-22, wobei die Polymerkomposition ein kovalent vernetztes Polymernetzwerk aufweist, welches durch eine nachträgliche Vernetzung von linearen oder verzweigten Polymeren gebildet wird.

24. Occlusionsinstrument gemäß Anspruch 23, wobei die Vernetzung durch ionisierende Strahlung oder durch thermische Spaltung radikal bildender Gruppe ausgelöst wird.

25. Occlusionsinstrument gemäß einem der vorhergehenden Ansprüche 9-24, wobei die Polymerkomposition zumindest ein biologisch abbaubares Material aufweist.

26. Occlusionsinstrument gemäß Anspruch 25, wobei die Polymerkomposition ein hydrolytisch abbaubares Polymer aufweist, insbesondere Poly(hydroxycarbonsäuren) bzw. entsprechende Copolymere.

27. Occlusionsinstrument gemäß Anspruch 25 oder 26, wobei die Polymerkomposition enzymatisch abbaubare Polymere aufweist.

28. Occlusionsinstrument gemäß einem der Ansprüche 25 bis 27, wobei die Polymerkomposition ein bioabbaubares thermoplastisches amorphes Polyurethan-Copolyester-Polymernetzwerk aufweist.

29. Occlusionsinstrument gemäß einem der Ansprüche 25 bis 28, wobei die Polymerkomposition ein bioabbaubares elastisches Polymernetzwerk aufweist, welches durch Vernetzung von oligomeren Diolen mit Diisocyanat erhalten werden.

30. Occlusionsinstrument gemäß einem der Ansprüche 25 bis 28, wobei die Polymerkomposition auf der Basis von kovalenten Netzwerken ausgehend von Oligo(ε-caprolacton)dimethacrylat und Butylacrylat gebildet ist.

31. Verfahren zur Herstellung eines Occlusionsinstrumentes gemäß einem der vorhergehenden Ansprüche, wobei ein kugelförmiges Hohlgeflecht derart hergestellt wird, dass dünne Drähte (7a) oder Fäden, welche das fertige Geflecht (7) begründen, beim Ausbilden des kugelförmigen Hohlgeflechts am distalen Ende (21) des Geflechts (7) untereinander verflochten werden, sodass ein distaler Retentionsbereich (3) eine zum distalen Ende (21) hin geschlossene Form aufweist.

32. Verfahren gemäß Anspruch 31, wobei zunächst das kugelförmige Hohlgeflecht aus einem einzigen Draht ausgebildet wird und die Enden des Drahtes ohne Fassung miteinander verbunden werden.

33. Verfahren gemäß Anspruch 31 oder 32, umfassend ausformen eines Retentionsbereiches und Mittenbereichs in einem einen Umformungs- und/oder Wärmebehandlungsschritt, wobei das kugelförmige Hohlgeflecht aus einem Material mit Formgedächtnis- oder Memory-Effekt besteht.

34. Verfahren gemäß Anspruch 33, wobei das Material eine Polymerkomposition ist, und wobei das Occlusionsinstrument zunächst durch konventionelle Verarbeitungsmethoden, wie etwa Spritzguss oder Extrusion, in eine permanente Form gebracht wird, und anschließend das Material deformiert wird und und in einer temporären Form fixiert wird.

## Claims

1. Occlusion instrument (150) comprising a mesh (7) consisting of at least one wire or thread (7a), where the occlusion instrument (150) has been suitably shaped by applying a forming and/or heat treatment method, is self-expandable, and configured to be safely anchored in an atrial appendage (10) of the left or right atrium of the heart, comprising a disc-shaped proximal retention area (2) at a proximal end (20) of the occlusion instrument(150) for occluding an orifice of the atrial appendage, and
where the occlusion instrument (150) comprises a closed distal end (21) without a hub,
**characterized by** an intermediate retention area (153) of the occlusion instrument (150), which has a substantially spherical, hollow shape, a distal retention area (3), an intermediate retention area (153), as well as a distal middle portion (155) located between the distal retention area (3) and the intermediate retention area (153), as well as a proximal middle portion (5) located between the proximal retention area (2) and the said intermediate retention area (153); where
the distal middle portion (155) of the occlusion instrument (150) has a cylindrically elongated shape and provides flexibility between the distal retention area (3) and the intermediate retention area (153).

2. Occlusion instrument (150) according to claim 1, where the at least one wire or thread converges into a socket in the proximal retention area (2).

3. Occlusion instrument (150) according to claim 1 or 2, where the occlusion instrument (150) is self-positioning and self-centering in the said atrial appendage of the left or right atrium.

4. Occlusion instrument (150) according to claim 1, with the proximal retention area (2) having a recess (8), in which a socket (4) is located, in which the ends of the wires or threads of the mesh (7) converge.

5. Occlusion instrument (150) according to any of the preceding claims, the occlusion instrument in its collapsed state having a diameter of 2 - 3.3 mm (6 - 10 French), rendering the surgery for occluding the atrial appendage is minimally invasive.

6. Occlusion instrument (150) according to any of the preceding claims, where the mesh (7) is a spherical wire mesh.

7. Occlusion instrument (150) according to claim 6, where the spherical wire mesh consists of multiple wires, and a proximal socket holds the ends of the wires together.

8. Occlusion instrument (150) according to claim 6, where the spherical wire mesh consists of a wire, and the ends of the wire are interconnected without a sleeve.

9. Occlusion instrument according to claims 1 to 8, where the threads of the mesh (7) consist of a shape memory polymer composition, so that the mesh (7) changes its shape from a temporary shape to a permanent shape under the influence of an external stimulus, where the temporary shape corresponds to the first shape of the mesh (7) and the permanent shape to the second shape of the mesh (7).

10. Occlusion instrument according to claim 9, where the external stimulus is a determinable switching temperature, with the switching temperature lying in the range between the room temperature and the patient's body temperature.

11. Occlusion instrument according to claim 10, where the polymer composition comprises polymeric switching elements, where below the determinable switching temperature the temporary shape of the mesh (7) is stabilized by using characteristic phase transitions of the polymeric switching elements.

12. Occlusion instrument according to claim 11, where the polymer composition comprises a crystalline or semi-crystalline polymer network with crystalline switching segments, with the temporary shape of the mesh (7) is being fixated and stabilized by freezing the crystalline switching segments at the crystallization transition, the switching temperature being determined by the crystallization temperature or the switching temperature of the crystalline switching segments.

13. Occlusion instrument according to claim 11, with the polymer composition comprising an amorphous polymer network with amorphous switching segments, where the temporary shape of the mesh (7) is fixated and stabilized by freezing the amorphous switching segments at the glass transition of the switching segments, the switching temperature being determined by the glass transition temperature of the amorphous switching segments.

14. Occlusion instrument according to one of the preceding claims 9 - 13, where the polymer composition has a linear, phase-segregated multi-block copolymer network, which can have at least two different phases, where the first phase is a hard segment forming phase, in which a plurality of hard segment forming blocks are formed in the polymer, which serve for a physical cross-linking of the polymeric structure and determine and stabilize the permanent shape of the mesh (7), and where the second phase is a switching segment forming phase, in which a plurality of switching segment forming blocks are formed in the polymer, which serve for fixating the temporary shape of the mesh (7), where the transition temperature from the switching segment forming phase to the hard segment forming phase is the switching temperature, and where, above the transition temperature of the hard segment forming phase, the shaping of the mesh (7) can be adjusted by applying conventional methods, particularly by applying injection molding or extrusion methods.

15. Occlusion instrument according to claim 14, where the polymer composition comprises thermoplastic polyurethane elastomers with a multi-block structure, with the hard segment forming phase being formed through a reaction of diisocyanates, particularly methylene-bis(4-phenylisocyanate) or hexamethylene diisocyanate, with dioles, particularly 1,4-butanediol, and where the switching segment forming phase results from oligomeric polyether or polyester dioles, particularly based on OH-terminated poly(tetrahydrofuran), poly(ε-caprolacetone), poly(ethyleneadipate), poly(ethyleneglyocol) or poly(propyleneglycol).

16. Occlusion instrument according to claim 14, with the polymer composition comprising phase-segregated diblock copolymers with an amorphous A-block and a semi-crystalline B-block, where the glass transition from the amorphous A-block constitutes the hard segment forming phase, and the melting temperature of the semi-crystalline B-block serves as a switching temperature for the thermal shape memory effect.

17. Occlusion instrument according to claim 16, with the polymer composition comprising polystyrene as the amorphous A-block and poly(1,4-butadiene) as the semi-crystalline B-block.

18. Occlusion instrument according to claim 14, the polymer composition comprising a phase-segregated triblock copolymer with a semi-crystalline central B-block and with two amorphous terminal A-blocks, where the A-blocks form the hard segment, and where the B-block determines the switching temperature.

19. Occlusion instrument according to claim 18, the polymer composition comprising semi-crystalline poly(tetrahydrofuran) as a central B-block and amorphous poly(2-methyloxazoline) as the terminal A-blocks.

20. Occlusion instrument according to one of the preceding claims 9 - 19, the polymer composition comprising polynorbornes, polyethylen-nylon-6-pfropfcopolymers and/or cross-linked poly(ethylen-co-vinylacetate)-copolymers.

21. Occlusion instrument according to one of the preceding claims 9 - 20, the polymer composition comprising a covalent cross-linked polymer network, which is formed by polymerisation, polycondensation and/or polyaddition of difunctional monomers or macromers with the addition of trifunctinal or higher polyfunctional crosslinkers, where the chemical, thermic and mechanical properties of the formed polymer network can be specifically adjusted by way of an appropriate selection of monomers as well as their functionality and share in the crosslinker.

22. Occlusion instrument according to claim 21, where the polymer composition is a covalent polymer network, which is created through cross-linking copolymerisation of stearyl acrylate and methacrylic acid with N,N'-methylenbisacrylamide as a crosslinker, with the shape memory effect of the polymer composition being based on crystallizing stearyl side chains.

23. Occlusion instrument according to one of the preceding claims 9 - 22, the polymer composition comprising a covalently cross-linked polymer network which is formed by way of a subsequent cross-linking of linear or branched polymers.

24. Occlusion instrument according to claim 23, with the cross-linking being initiated by ionizing radiation or by thermal cracking of radical-forming groups.

25. Occlusion instrument according to one of the preceding claims 9 - 24, the polymer composition comprising at least one biodegradable material.

26. Occlusion instrument according to claim 25, the polymer composition comprising a hydrolytically degradable polymer, particularly poly(hydroxycarboxylic acids) or respective copolymers.

27. Occlusion instrument according to claim 25 or 26, the polymer composition comprising encymatically degradable polymers.

28. Occlusion instrument according to any of the claims 25 to 27, the polymer composition comprising a biodegradable thermoplastic amorphous polyurethane-copolyester-polymer network.

29. Occlusion instrument according to any of the claims 25 bis 28, the polymer composition comprising a biodegradable elastic polymer network, which results from cross-linking oligomeric diols with diisocyanate.

30. Occlusion instrument according to any of the claims 25 to 28, with the polymer composition being formed based on covalent networks based on oligo(ε-caprolactone)dimethacrylate and butylacrylate.

31. Method for manufacturing an occlusion instrument according to one of the preceding claims, where the spherical hollow mesh is made in such a way that thin wires (7a) or threads which constitute the finished mesh (7) are intertwined at the distal end (21) of the mesh (7) during the forming procedure of the spherical hollow mesh, so that a distal retention area (3) has a shape that is closed towards the distal end (21) .

32. Method according to claim 31, where initially the spherical hollow mesh is formed from a single wire and the ends of the wire are connected with each other without a socket.

33. Method according to claim 31 or 32, comprising the formation of a retention area and a middle portion in a forming and/or heat treatment step, with the spherical hollow mesh consisting of a material with a shape memory or memory effect.

34. Method according to claim 33, where the material is a polymer composition, and where the occlusion instrument is initially brought into a permanent shape by applying conventional processing methods, such as injection molding or extrusion, and subsequently the material is deformed and fixated in a temporary shape.

## Revendications

1. Dispositif d'occlusion (150) composé d'un tressage (7) d'au moins un fil métallique ou fil (7a), où le dispositif d'occlusion (150) s'est vu donner une forme appropriée dans un procédé de mise en forme et/ou de traitement thermique, peut s'auto-déployer et est configuré pour un ancrage sûr dans une auricule cardiaque (10) de l'oreillette gauche ou droite d'un cœur, comprenant une zone de retenue en forme de disque (2) au niveau d'une extrémité proximale (20) du dispositif d'occlusion (150) pour l'occlusion de l'ouverture de l'auricule cardiaque,
et où le dispositif d'occlusion (150) présente une extrémité distale fermée(21) sans moyeu,
**caractérisé par** une zone de retenue intermédiaire (153) du dispositif d'occlusion (150), laquelle est de forme sensiblement sphérique et creuse, une zone de retenue distale (3), une zone de retenue intermédiaire (153) ainsi que par une zone centrale distale (155), disposée entre la zone de retenue distale (3) et la zone de retenue intermédiaire (153), ainsi que par une zone centrale proximale (5) entre la zone de retenue proximale (2) et ladite zone de retenue intermédiaire (153); où
la zone centrale distale (155) du dispositif d'occlusion (150) est de forme cylindrique allongée et assure la flexibilité de la zone de retenue distale (3) par rapport à la zone de retenue intermédiaire (153).

2. Dispositif d'occlusion (150) selon la revendication 1, où au moins un fil métallique ou fil est relié à un support dans la zone de retenue proximale (2).

3. Dispositif d'occlusion (150) selon l'une des revendications 1 et 2, où le dispositif d'occlusion (150) peut s'auto-positionner et s'auto-centrer dans ladite auricule cardiaque de l'oreillette gauche ou droite.

4. Dispositif d'occlusion (150) selon la revendication 1, où la zone de retenue proximale (2) présente un creux (8) dans lequel se trouve un support (4) dans lequel les bouts des fils métalliques ou des fils du tressage (7) sont reliés.

5. Dispositif d'occlusion (150) selon l'une des revendications précédentes, où le dispositif d'occlusion présente un diamètre de 2 à 3,3 mm (6 à 10 unités Charrière) à l'état replié, de sorte que l'intervention d'occlusion de l'auricule cardiaque soit peu invasive.

6. Dispositif d'occlusion (150) selon l'une des revendications précédentes, où le tressage (7) est un tressage sphérique de fil métallique.

7. Dispositif d'occlusion (150) selon la revendication 6, où le tressage sphérique de fil métallique est composé de plusieurs fils et où le support proximal relie les extrémités des fils.

8. Dispositif d'occlusion (150) selon la revendication 6, où le tressage sphérique de fil métallique est composé d'un fil et où les extrémités du fil sont liées entre elles sans garde.

9. Dispositif d'occlusion selon les revendications 1 à 8, où les fils du tressage (7) sont constitués d'une composition polymère à mémoire de forme, de sorte que le tressage, sous l'effet d'un stimulus externe, passe d'une forme temporaire à une forme permanente, où la forme temporaire correspond à la première mise en forme du tressage (7) et la forme permanente à la deuxième mise en forme du tressage (7).

10. Dispositif d'occlusion selon la revendication 9, où le stimulus externe est une température de transformation pouvant être fixée, où la température de transformation se situe entre la température ambiante et la température corporelle du patient.

11. Dispositif d'occlusion selon la revendication 10, où la composition polymère présente des éléments de transformation polymères, où la forme temporaire du tressage (7) est stabilisée en dessous de la température de transformation pouvant être fixée à l'aide des transitions de phase caractéristiques des éléments de transformation polymères.

12. Dispositif d'occlusion selon la revendication 11, où la composition polymère présente un réseau polymère cristallin ou partiellement cristallin comportant des segments de transformation cristallins, où la forme temporaire du tressage (7) est fixée et stabilisée par congélation des segments de transformation cristallins lors d'une transition de cristallisation, où la température de transformation est déterminée par la température de cristallisation ou la température de transformation des segments de transformation cristallins.

13. Dispositif d'occlusion selon la revendication 11, où la composition polymère présente un réseau polymère amorphe comportant des segments de transformation amorphes, où la forme temporaire du tressage (7) est fixée et stabilisée par congélation des segments de transformation amorphes lors d'une transition vitreuse des segments de transformation, où la température de transformation est déterminée par la température de transition vitreuse des segments de transformation amorphes.

14. Dispositif d'occlusion selon l'une des revendications précédentes 9 à 13, où la composition polymère présente un réseau de copolymère multibloc linéaire séparé en phases, qui peut se présenter selon au moins deux phases différentes, où la première phase est une phase de formation de segments durs dans laquelle une multitude de blocs de formation de segments durs sont formés dans le polymère, lesquels servent à la réticulation physique de la structure polymère et déterminent et stabilisent la forme permanente du tressage (7), et où la deuxième phase est la phase de formation de segments de transformation dans laquelle une multitude de blocs de formation de segments de transformation sont formés dans le polymère, lesquels servent à la fixation de la forme temporaire du tressage (7), où la température de transition de la phase de formation de segments de transformation en la phase de formation de segments durs correspond à la température de transformation, et où au-dessus de la température de transition de la phase formation de segments durs, la mise en forme du tressage (7) peut être ajustée par des procédés conventionnels, en particulier par des procédés de moulage par injection ou d'extrusion.

15. Dispositif d'occlusion selon la revendication 14 où la composition polymère présente des élastomères de polyuréthane thermoplastiques ayant une structure multibloc où la phase de formation de segments durs est formée par une transformation des diisocyanates, notamment du méthylène bis(4-phénylisocyanate) ou de l'hexaméthylènediisocyanate, par des diols, notamment du 1,4-butanediol, et où la phase de formation de segments de transformation résulte de polyéthers ou polyesterdiols oligomères, notamment du poly(tétrahydrofurane), de la poly(ε-caprolactone), du poly(adipate d'éthylène), du poly(éthylèneglycol) ou du poly(propylèneglycol) à groupement terminal OH.

16. Dispositif d'occlusion selon la revendication 14, où la composition polymère présente des copolymères diblocs séparés en phases avec un bloc A amorphe et un bloc B partiellement cristallin, où la transition vitreuse du bloc A amorphe forme la phase de formation de segments durs, et où la température de fusion du bloc B partiellement cristallin sert de température de transformation à l'effet thermique de mémoire de forme.

17. Dispositif d'occlusion selon la revendication 16, où la composition polymère présente du polystyrène en tant que bloc A amorphe et du poly(1,4-butadiène) en tant que bloc B partiellement cristallin.

18. Dispositif d'occlusion selon la revendication 14 où la composition polymère présente un copolymère tribloc séparé phases avec un bloc B central partiellement cristallin et deux blocs A terminaux amorphes où les blocs A forment le segment dur, et où le bloc B définit la température de transformation.

19. Dispositif d'occlusion selon la revendication 18, où la composition polymère présente du poly(tétrahydrofurane) partiellement cristallin en tant que bloc B central et de la poly(2-méthyloxazoline) amorphe en tant que blocs A terminaux.

20. Dispositif d'occlusion selon l'une des revendications précédentes 9 à 19, où la composition polymère présente du polynorbornène, un copolymère greffé de polyéthylène-nylon-6 et/ou un copolymère de poly(éthylène-co-vinylacétate) réticulé.

21. Dispositif d'occlusion selon l'une des revendications précédentes 9 à 20, où la composition polymère présente un réseau polymère réticulé covalent qui est formé par polymérisation, polycondensation et/ou polyaddition de monomères ou macromères difonctionnels grâce à l'ajout d'agents de réticulation trifonctionnels ou ayant plus de fonctions, où un choix approprié des monomères, de leurs fonctionnalités et de leur proportion dans les agents de réticulation permet d'ajuster de façon sélective les propriétés chimiques, thermiques et mécaniques du réseau polymère formé.

22. Dispositif d'occlusion selon la revendication 21, où la composition polymère est un réseau polymère covalent formé, qui est formé par copolymérisation par réticulation du stéarylacrylate et de l'acide méthacrylique avec du N,N'-méthylène-bis-acrylamide en tant qu'agent de réticulation, où l'effet de mémoire de forme de la composition polymère résulte des chaînes latérales de stéaryle cristallisantes.

23. Dispositif d'occlusion selon l'une des revendications précédentes 9 à 22, où la composition polymère présente un réseau polymère réticulé covalent, qui est formé par une réticulation ultérieure de polymères linéaires ou ramifiés.

24. Dispositif d'occlusion selon la revendication 23, où la réticulation est déclenchée par un rayonnement ionisant ou une décomposition thermique de groupes formant des radicaux.

25. Dispositif d'occlusion selon l'une des revendications précédentes 9 à 24, où la composition polymère présente au moins un matériau biodégradable.

26. Dispositif d'occlusion selon la revendication 25, où la composition polymère présente un polymère dégradable par voie hydrolytique, notamment des acides poly(hydroxycarboxyliques) ou des copolymères correspondants.

27. Dispositif d'occlusion selon la revendication 25 ou 26, où la composition polymère présente des polymères dégradables par voie enzymatique.

28. Dispositif d'occlusion selon l'une des revendications 25 à 27 où la composition polymère présente un réseau polymère de polyuréthane-copolyester biodégradable, thermoplastique et amorphe.

29. Dispositif d'occlusion selon l'une des revendications 25 à 28, où la composition polymère présente un réseau polymère biodégradable et élastique, qui est obtenu par la réticulation de diols oligomères avec du diisocyanate.

30. Dispositif d'occlusion selon l'une des revendications 25 à 28, où la composition polymère est formée sur la base de réseaux covalents à partir d'oligo(ε-caprolactone)diméthylacrylate et de butylacrylate.

31. Procédé de fabrication d'un dispositif d'occlusion selon l'une des revendications précédentes où un tressage sphérique creux est fabriqué de façon à ce que les fils métalliques fins (7a) ou les fils formant le tressage fini (7) soient tressés entre eux lors de la formation du tressage sphérique creux sur l'extrémité distale (21) du tressage (7) de sorte qu'une zone de retenue distale (3) présente une forme fermée vers l'extrémité distale (21).

32. Procédé selon la revendication 31, où le tressage sphérique creux est d'abord formé par un seul fil métallique et les extrémités du fil métallique sont liées entre elles sans support.

33. Procédé selon la revendication 31 ou 32, comprenant la mise en forme d'une zone de retenue et d'une zone centrale dans une étape de mise en forme et/ou de traitement thermique, où le tressage sphérique creux est dans un matériau à mémoire de forme ou à effet mémoire.

34. Procédé selon la revendication 33, où le matériau est une composition polymère, et où le dispositif d'occlusion se voit donner une forme permanente par des méthodes de travail conventionnelles telles qu'un moulage par injection ou une extrusion, puis le matériau est déformé et est fixé dans une forme temporaire.
